(19) Europäisches Patentamt
European Patent Office
Office européen des brevets

(11) **EP 3 706 900 B1**

(12) **EUROPÄISCHE PATENTSCHRIFT**

(45) Veröffentlichungstag und Bekanntmachung des Hinweises auf die Patenterteilung:
**07.12.2022 Patentblatt 2022/49**

(21) Anmeldenummer: **18795645.3**

(22) Anmeldetag: **31.10.2018**

(51) Internationale Patentklassifikation (IPC):
**B01J 20/26** (2006.01)  **B01J 20/02** (2006.01)
**B01J 20/30** (2006.01)  **B01J 20/32** (2006.01)
**C08F 220/06** (2006.01)  **A61L 15/24** (2006.01)

(52) Gemeinsame Patentklassifikation (CPC):
(C-Sets verfügbar)
**B01J 20/267; A61L 15/24; A61L 15/60;
B01J 20/0248; B01J 20/0292; B01J 20/3007;
B01J 20/3021; B01J 20/3078; B01J 20/321;
B01J 20/3236; C08F 8/14; C08F 8/44;
C08F 220/06;** B01J 2220/68        (Forts.)

(86) Internationale Anmeldenummer:
**PCT/EP2018/079858**

(87) Internationale Veröffentlichungsnummer:
**WO 2019/091848 (16.05.2019 Gazette 2019/20)**

(54) **SUPERABSORBER**

SUPER ABSORBER

MATÉRIAU SUPERABSORBANT

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorität: **10.11.2017 EP 17200963**

(43) Veröffentlichungstag der Anmeldung:
**16.09.2020 Patentblatt 2020/38**

(73) Patentinhaber: **BASF SE**
**67056 Ludwigshafen am Rhein (DE)**

(72) Erfinder:
• **HARTNAGEL, Kristine**
**67056 Ludwigshafen (DE)**
• **POOMSUWAN, Wanthip**
**Chonburi 20000 (TH)**
• **HAMILTON, Patrick Neal**
**Freeport, Texas 77541 (US)**
• **HERFERT, Norbert**
**Shanghai 200137 (CN)**

• **DANIEL, Thomas**
**67056 Ludwigshafen (DE)**
• **HOELLER, Olaf**
**Charlotte, North Carolina 28273 (US)**

(74) Vertreter: **BASF IP Association**
**BASF SE**
**GBI-C006**
**67056 Ludwigshafen (DE)**

(56) Entgegenhaltungen:
**EP-A1- 2 163 302        WO-A1-2008/092842
WO-A1-2011/113777      WO-A2-2010/012762
WO-A2-2011/061125**

(52) Gemeinsame Patentklassifikation (CPC): (Forts.)

C-Sets
**C08F 8/14, C08F 220/06;**
**C08F 8/44, C08F 220/06**

C-Sets
**C08F 8/14, C08F 220/06;**
**C08F 8/44, C08F 220/06**

**Beschreibung**

Die vorliegende Erfindung betrifft einen verbesserten Superabsorber, ein Verfahren zu seiner Herstellung sowie seine Verwendung und ihn enthaltende Hygieneartikel.

Superabsorber sind bekannt. Für derartige Materialien sind auch Bezeichnungen wie "hochquellfähiges Polymer" "Hydrogel" (oft auch für die trockene Form verwendet), "Hydrogel bildendes Polymer", "Wasser absorbierendes Polymer", "absorbierendes gelbildendes Material", "quellfähiges Harz", "wasserabsorbierendes Harz" oder ähnliche gebräuchlich. Es handelt sich dabei um vernetzte hydrophile Polymere, insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboximethylcellulose, teilweise vernetztes Polyalkylenoxid oder in wässrigen Flüssigkeiten quellbare Naturprodukte, wie beispielsweise Guarderivate, wobei wasserabsorbierende Polymere auf Basis teilneutralisierter Acrylsäure am weitesten verbreitet sind. Die wesentlichen Eigenschaften von Superabsorbern sind ihre Fähigkeiten, ein Vielfaches ihres Eigengewichts an wässrigen Flüssigkeiten zu absorbieren und die Flüssigkeit auch unter gewissem Druck nicht wieder abzugeben. Der Superabsorber, der in Form eines trockenen Pulvers eingesetzt wird, wandelt sich bei Flüssigkeitsaufnahme in ein Gel, bei der üblichen Wasseraufnahme entsprechend in ein Hydrogel um. Die Vernetzung ist für synthetische Superabsorber wesentlich und ein wichtiger Unterschied zu üblichen reinen Verdickern, da sie zur Unlöslichkeit der Polymeren in Wasser führt. Lösliche Substanzen wären als Superabsorber nicht brauchbar. Das mit weitem Abstand wichtigste Einsatzgebiet von Superabsorbern ist das Absorbieren von Körperflüssigkeiten. Superabsorber werden beispielsweise in Windeln für Kleinkinder, Inkontinenzprodukten für Erwachsene oder Damenhygieneprodukten verwendet. Andere Anwendungsgebiete sind beispielsweise die als Wasser zurückhaltende Mittel im landwirtschaftlichen Gartenbau, als Wasserspeicher zum Schutz vor Feuer, zur Flüssigkeitsabsorption in Lebensmittelverpackungen oder ganz allgemein zur Absorption von Feuchtigkeit.

Superabsorber können ein Mehrfaches ihres Eigengewichts an Wasser absorbieren und unter gewissem Druck zurückhalten. Im Allgemeinen weist ein derartiger Superabsorber eine CRC ("Centrifuge Retention Capacity", Messmethode siehe unten) von mindestens 5 g/g, vorzugsweise mindestens 10 g/g und in besonders bevorzugter Form mindestens 15 g/g auf. Ein "Superabsorber" kann auch ein Gemisch stofflich verschiedener einzelner Superabsorber sein oder ein Gemisch von Komponenten, die erst im Zusammenwirken superabsorbierende Eigenschaften zeigen, es kommt hier weniger auf die stoffliche Zusammensetzung an als auf die superabsorbierenden Eigenschaften.

Wichtig für einen Superabsorber ist nicht nur seine Absorptionskapazität, sondern auch die Fähigkeit, Flüssigkeit unter Druck zurückzuhalten (Retention, meist als "Absorption unter Druck", "Absorption under Load" ("AUL") oder "Absorption against Pressure" ("AAP") ausgedrückt, Messmethode siehe unten) sowie die Permeabilität, also die Fähigkeit zur Flüssigkeitsweiterleitung im gequollenen Zustand (meist als "Saline Flow Conductivity" ("SFC") oder als "Gel Bed Permeability" ("GBP") ausgedrückt, Messmethoden siehe unten (wobei Veränderungen am Superabsorber seine SFC und GBP-Werte nicht unbedingt beide oder beide gleichermaßen verändern)). Gequollenes Gel kann die Flüssigkeitsweiterleitung zu noch nicht gequollenem Superabsorber behindern bis verhindern ("gel blocking"). Gute Weiterleitungseigenschaften für Flüssigkeiten besitzen beispielsweise Hydrogele, die im gequollenen Zustand eine hohe Gelfestigkeit aufweisen. Gele mit nur geringer Gelfestigkeit sind unter einem angewendeten Druck (Körperdruck) deformierbar, verstopfen Poren in dem Superabsorber / Cellulosefaser-Saugkörper und verhindern dadurch die Flüssigkeitsweiterleitung zu noch nicht oder nicht vollständig gequollenem Superabsorber und die Flüssigkeitsaufnahme durch diesen noch nicht oder nicht vollständig gequollenen Superabsorber. Eine erhöhte Gelfestigkeit wird in aller Regel durch einen höheren Vernetzungsgrad erreicht, wodurch allerdings die Absorptionskapazität des Produktes verringert wird. Eine elegante Methode zur Erhöhung der Gelfestigkeit stellt die Erhöhung des Vernetzungsgrads an der Oberfläche der Superabsorberpartikel gegenüber dem Inneren der Partikel dar. Dazu werden meist in einem Oberflächennachvernetzungsschritt getrocknete Superabsorberpartikel mit durchschnittlicher Vernetzungsdichte einer zusätzlichen Vernetzung in einer dünnen Oberflächenschicht ihrer Partikel unterworfen. Durch die Oberflächennachvernetzung steigt die Vernetzungsdichte in der Schale der Superabsorberpartikel, wodurch die Absorption unter Druckbelastung auf ein höheres Niveau gehoben wird. Während die Absorptionskapazität in der Oberflächenschicht der Superabsorberpartikel sinkt, weist ihr Kern durch das Vorhandensein beweglicher Polymerketten eine im Vergleich zur Schale verbesserte Absorptionskapazität auf, so dass durch den Schalenaufbau eine verbesserte Permeabilität gewährleistet wird, ohne dass gel blocking auftritt. Es ist ebenfalls bekannt, insgesamt höher vernetzte Superabsorber zu erzeugen und den Vernetzungsgrad im Inneren der Partikel gegenüber einer äußeren Schale der Partikel nachträglich zu verringern.

Auch Verfahren zur Herstellung von Superabsorbern sind bekannt. Superabsorber auf Basis von Acrylsäure, die auf dem Markt am gängigsten sind, werden durch radikalische Polymerisation von Acrylsäure in Gegenwart eines Vernetzers (dem "Innenvernetzer") hergestellt, wobei die Acrylsäure vor, nach oder teils vor, teils nach der Polymerisation zu einem gewissen Grad neutralisiert wird, üblicherweise durch Zugabe von Alkali, meist einer wässrigen Natriumhydroxidlösung. Das so gewonnene Polymergel wird zerkleinert (je nach verwendetem Polymerisationsreaktor kann dies gleichzeitig mit der Polymerisation erfolgen) und getrocknet. Das so gewonnene trockene Pulver (das "Grundpolymer" oder "Basispolymer") wird üblicherweise an der Oberfläche der Partikel nachvernetzt, indem es mit weiteren Vernetzern wie etwa organischen Vernetzern oder mehrwertigen Kationen, beispielsweise Aluminium (meist als Aluminiumsulfat eingesetzt) oder beidem umgesetzt wird, um eine gegenüber dem Partikelinneren stärker vernetzte Oberflächenschicht zu erzeugen.

Ein bei Superabsorbern oft auftretendes Problem sind Verfärbungen, die beim Lagern unter höherer Temperatur oder höherer Luftfeuchtigkeit auftreten. Derartige Bedingungen treten oft bei Lagerung von Superabsorbern in tropischen oder subtropischen Ländern auf. Unter solchen Bedingungen neigen Superabsorber zum Vergilben, sie können sogar braune oder gar fast schwarze Färbung annehmen. Diese Verfärbung des eigentlich farblosen Superabsorberpulvers ist unansehnlich und unerwünscht, da sie insbesondere bei den erwünschten dünnen Hygieneprodukten sichtbar ist und Verbraucher unansehnliche Hygieneprodukte ablehnen. Die Ursache für die Verfärbung ist nicht vollständig geklärt, allerdings scheinen reaktive Verbindungen wie Restmonomere aus der Polymerisation, die Verwendung mancher Initiatoren, Verunreinigungen des Monomeren oder des Neutralisationsmittels, Oberflächennachvernetzer oder Stabilisatoren der verwendeten Monomere eine Rolle zu spielen.

Fredric L. Buchholz und Andrew T. Graham (Hrsg.) geben in: "Modern Superabsorbent Polymer Technology", J. Wiley & Sons, New York, U.S.A. / Wiley-VCH, Weinheim, Germany, 1997, ISBN 0-471-19411-5, einen zusammenfassenden Überblick über Superabsorber, ihre Eigenschaften und Verfahren zur Herstellung von Superabsorbern.

Der Zusatz mehrwertiger Kationen zu Superabsorbern im Zuge der Oberflächennachvernetzung mit kovalente Bindungen zwischen den Polymerketten ausbildenden Oberflächennachvernetzungsmitteln ist bekannt. So beschreibt WO 98/48 857 A1 Superabsorber, die mit Al-, Fe-, Zr-, Mg- oder Zn-Kationen vernetzt und dann mit einer Flüssigkeit wie Wasser, Mineralöl oder Polyolen versetzt werden. WO 01/74 913 A1 betrifft die Regenerierung von Superabsorbern, spezifisch die Erhöhung einer durch Abrieb verringerten Permeabilität, durch Zugabe einer Lösung eines wenigstens dreiwertigen Kations, typischerweise einer wässrigen Aluminiumsulfatlösung. US 6 620 889 B1 offenbart Superabsorber, die mit einer Kombination aus einem Polyol und einem Salz eines mehrwertigen Metalls in wässriger Lösung oberflächennachvernetzt werden. Das Anion des Salzes kann Chlorid, Bromid, Sulfat, Carbonat, Nitrate, Phosphat, Acetat oder Laktat sein. Die Verwendung von Aluminiumsulfat ist bevorzugt.

Nach der Lehre von WO 2006/111 402 A2 wird ein Grundpolymer mit einem Permeabilitätsverbesserer behandelt, der aus Silicium-Sauerstoff-Verbindungen, Salzen mehrwertiger, insbesondere dreiwertiger Kationen oder Mischungen davon gewählt wird. Das Salz eines dreiwertigen Kations ist vorzugsweise ein Aluminiumsalz, das aus einer Reihe von Salzen gewählt wird, die Aluminium-Laktat, -Oxalat, -Citrat, -Glyoxylat, -Succinat, -Tartrat und andere organische und anorganische Aluminiumsalze einschließt. WO 2005/108 472 A1 offenbart ein Verfahren, das die Behandlung eines Grundpolymers mit einem wasserlöslichen Salz eines mehrwertigen Metalls und einer organischen Säure oder ihrem Salz umfasst. Das Salz eines mehrwertigen Metalls ist vorzugsweise Aluminiumsulfat. Die organische Säure oder ihr Salz wird aus einer Reihe von Säuren gewählt, die Citronensäure, Glyoxylsäure, Glutarsäure, Bernsteinsäure, Weinsäure, Milchsäure sowie die Alkali- oder Ammoniumsalze dieser Säuren einschließt.

WO 2004/113 452 A1 beschreibt Superabsorber, die mit konzentrierten Lösungen von mehrwertigen Metallsalzen, insbesondere Natriumaluminiumat behandelt werden.

WO 2013/156 281 A1 lehrt die Behandlung von Superabsorbern mit Aluminiumglycinat.

WO 2010/108 875 A1, WO 2012/045 705 A1 und WO 2013/156 330 A1 lehren die Behandlung von Superabsorbern mit basischen Aluminiumsalzen wie basischem Aluminiumacetat oder Aluminiumlaktat.

WO 2009/080 611 A2 offenbart die Behandlung von Superabsorbern mit Mischungen von Aluminiumsalzen, von denen eines ein chelatisierendes Anion, beispielsweise Dicarboxylate oder Hydroxicarboxylate enthält, wobei Laktat besonders bevorzugt ist, und das andere ein schwach komplexierendes Anion, beispielsweise Chlorid, Nitrat, Sulfat, Hydrogensulfat, Carbonat, Hydrogencarbonat, Nitrat, Phosphat, Hydrogenphosphat, Dihydrogenphosphat oder Carboxylat, wobei Sulfat besonders bevorzugt ist.

Es ist unbekannt, wie genau die in den Aluminiumsalzen verwendeten Anionen letztlich auf dem Superabsorber vorliegen. Anders gesagt, die genaue Struktur der auf den Partikeloberflächen entstehenden Aluminiumkomplexe ist unbekannt. Aus dem Stand der Technik ist jedoch bekannt, dass die in den Aluminiumsalzen verwendeten Anionen die Eigenschaften des Superabsorbers beeinflussen. Ein Problem bei der Verwendung bestimmter Anionen oder bestimmter Anionengemische ist deren Löslichkeit in der zumeist wässrigen Aluminiumsalzlösung, die auf den Superabsorber aufgebracht wird. Fallen dort Verbindungen aus, ist also die Lösung nicht stabil, entstehen je nach den Umständen der Zugabe, insbesondere Alter und Temperatur der Lösung, uneinheitliche Superabsorber und Aluminium wird als fester Niederschlag der Komplexierung von Carboxylatgruppen an der Oberfläche der Superabsorberpartikel entzogen, so dass die gewünschte permeabilitätsteigernde Wirkung nicht oder nur vermindert eintritt. Beispielsweise ist aufgrund der niedrigen Löslichkeit von Aluminiumphosphat Phosphat bisher ein bei der Komplexierung von Superabsorbern mit Aluminium nur schwer verwendbares Anion. Es mag Gründe geben, einen Superabsorber mit festem oder suspendiertem Aluminiumphosphatpulver zu versetzen, dies ist jedoch keine Oberflächenkomplexierung im Interesse höherer Permeabilität.

Zur Stabilisierung von Superabsorbern gegen Verfärbung wird oft ein Reduktionsmittel zugegeben. WO 00/55 245 A1 lehrt die Stabilisierung von Superabsorbern gegen Verfärbung durch Behandlung mit einem anorganischen Reduktionsmittel und wahlweise einem Metallsalz wie etwa einem Erdalkalisalz, das nach der Polymerisation zugegeben wird. Das anorganische Reduktionsmittel ist typischerweise ein Hypophosphit, Phosphit, Bisulfit oder Sulfit. Das Metallsalz ist typischerweise ein farbloses (die Eigenschaft "farblos" wird oft auch einfach "weiß" genannt) Phosphat, Acetat oder Lactat, aber kein Halogenid. Nach der Lehre von WO 2006/058 682 A1 werden Verfärbungen von Superabsorbern vermieden, wenn die Trocknung und die Nachvernetzungsreaktion in einer Atmosphäre durchgeführt werden, die im Wesentlichen frei von oxidierenden Gasen ist. WO 2009/060 062 A1 oder WO 2010/012 762 A2 lehren den Zusatz von Sulfinsäurederivaten zu Superabsorbern, um diese gegen Verfärbung zu stabilisieren. EP 1 199 315 A2 lehrt die Verwendung eines Redoxinitiatorsystems zur Initiierung einer Polymerisationsreaktion, wobei das Redoxinitiatorsystem als reduzierenden Teil eine Sulfinsäure oder ein Sulfinat, insbesondere 2-Hydroxisulfinatoessigsäure oder ein Salz davon enthält. WO 99/18 067 A1 offenbart bestimmte Hydroxi- oder Aminoalkyl- oder aryl-sulfinsäurederivate oder Gemische davon und ihre Verwendung als Reduktionsmittel, die keinen Formaldehyd abspalten. WO 2004/084 962 A1 betrifft die Verwendung dieser Sulfinsäurederivate als reduzierenden Teil eines Redoxinitiators zur Polymerisation teilneutralisierter Acrylsäure zu Superabsorbern.

Die Offenlegungsschrift JP 05/086 251 lehrt die Verwendung von Phosphorsäurederi-vaten oder Salzen davon, insbesondere (1-Hydroxiethan-1,1-diyl)bisphosphonsäure (auch "1-Hydro-xiethyliden-1,1-diphosphonsäure", "1-Hydroxiethan-(1,1-diphosphonsäure)", Trivialname "Etidronsäure"), Ethylendiamintet-ra(methylenphosphonsäure), Diethylentriaminpenta(methylen-phosphonsäure) oder deren Alkali- oder Ammoniumsalze als Stabilisatoren von Superabsorbern gegen Verfärbung. EP 781 804 A2 lehrt zum gleichen Zweck die Zugabe von (1-Hydroxialkyl-1,1-diyl)bisphosphonsäuren, wobei der Alkylrest von 5 bis zu 23 Kohlenstoffatome um-fasst. EP 668 080 A2 lehrt den Zusatz anorganischer Säuren, organischer Säuren oder Poly-aminosäuren zu Superabsorbern, wobei unter den angegebenen anorganischen Säuren auch auf Phosphor basierende Säuren sind. US 2005/0 085 604 A1 offenbart den Zusatz von Chelatbildnern sowie Oxidations- oder Reduktionsmitteln zu Superabsor-bern, wobei unter den Chelatbildnern auch Phosphor enthaltende sind. US 2005/0 272 600 A1 betrifft die Zugabe von Ionenblockern zu Superabsorbern, zu denen auch organische Phosphorverbindungen gehören. (1-Hydroxiethan-1,1-diyl)bisphosphonsäure ist eines der genannten Beispiele. Nach der Lehre von EP 2 112 172 A1 wird der Monomerlösung, die zum Superabsorber polymerisiert wird, eine organische Phosphorverbindung zugesetzt, (1-Hydroxiethan-1,1-diyl)bisphosphonsäure wird genannt, Ethylendiamintetra(methylenphosphonsäure) ist die bevorzugteste Verbindung. US 2009/0 275 470 A1 lehrt, Superabsorbern sowohl Chelatbildner als auch vorzugsweise anorganische Phosphorverbindungen zuzugeben, wobei der Chelatbildner auch eine Phosphorverbindung wie beispielsweise (1-Hydroxiethan-1,1-diyl)bisphosphonsäure oder Ethylendiamin-tetra(methylenphosphonsäure) sein kann. Derartige Verbindungen werden auch nach der Lehre von WO 2006/109 882 A1 Superabsorbern als Chelatbildner zugesetzt, wobei neben phosphorhaltigen Verbindungen auch schwefelhaltige Reduktionsmittel in verschiedenen Verfahrensstufen eingesetzt werden. WO 2013/144 026 A1 lehrt Superabsorber, die mit Alumininiumionen und Etidronsäure beschichtet sind, die in einem bestimmten molaren Verhältnis stehen.

EP2163302 A1 offenbart ebenfalls einen Superabsorber der mit Aluminiumionen komplexiert wird.

Es besteht daher weiterhin die Aufgabe, andere oder sogar verbesserte, insbesondere sowohl permeable als auch gegen Verfärbung, insbesondere gegen Vergilbung oder Braunfärbung bei Lagerung unter erhöhter Temperatur und/oder erhöhter Luftfeuchtigkeit stabilisierte Superabsorber sowie Verfahren zu ihrer Herstellung zu finden. Diese Superabsorber sollten auch ohne Niederschlagsprobleme bei der Komplexierung mit Aluminiumionen insbesondere in Anwesenheit von Phosphationen hergestellt werden können. Die Gebrauchseigenschaften des Superabsorbers, insbesondere seine Aufnahmefähigkeit für Flüssigkeit, auch unter Druck, sollen dabei nicht oder zumindest nicht wesentlich beeinträchtigt werden. Weitere Aufgaben der Erfindung sind Verwendungen dieses Superabsorbers, wie Hygieneprodukte, die diesen Superabsorber enthalten und Verfahren zu deren Herstellung.

Gelöst wurde die Aufgabe durch einen mit Aluminiumionen komplexierten Superabsorber, wobei die Aluminiumionen in Form einer wässrigen, Aluminiumionen enthaltenden Lösung aufgebracht werden, die sich dadurch auszeichnet, dass sie Aluminiumionen in einem Anteil im Bereich von 0,5 - 15 Gewichts-% (ggf. umgerechnet auf $Al^{3+}$) bezogen auf die Gesamtmasse der Lösung umfasst und weiterhin Anionen der Milchsäure (Laktat-Ionen) und der Phosphorsäure (Phosphat-Ionen) umfasst, wobei der molare Anteil der Laktat-Ionen im Bereich des 0,01 - 2,99-fachen bezogen auf den molaren Anteil an $Al^{3+}$ ist und der molare Anteil der Phosphat-Ionen im Bereich des 0,01 - 2,99-fachen bezogen auf den molaren Anteil an $Al3^+$ ist.

Mengenanteile für die mit Ladung angegebenen Ionen beziehen sich dabei jeweils auf die Ionen selbst, so dass beispielsweise als Oxide (z.B. $Al_2O_3$) bestimmte oder dazu umgerechnete Mengenanteile auf die Ionen umzurechnen sind.

Ferner wurde ein Verfahren zur Herstellung des erfindungsgemäßen Superabsorbers gefunden, nämlich durch Polymerisation einer wässrigen Monomerlösung, die

a) mindestens ein ethylenisch ungesättigtes, säuregruppentragendes Monomer, das wahlweise zumindest teilweise als Salz vorliegt,
b) mindestens einen Vernetzer,

c) mindestens einen Initiator,

d) wahlweise ein oder mehrere mit den unter a) genannten Monomeren copo-lymerisierbare ethylenisch ungesättigte Monomere,

e) wahlweise ein oder mehrere wasserlösliche Polymere;

enthält, wobei das Verfahren weiterhin

Trocknung des erhaltenen Polymerisats,

wahlweise Mahlung des getrockneten Polymerisats und Siebung des gemahlenen Polymerisats,

wahlweise Oberflächennachvernetzung des getrockneten und gegebenenfalls gemahlenen und gesiebten Polymerisats, und

Zugabe einer wässrigen, Aluminiumionen enthaltenden Lösung, die sich dadurch auszeichnet, dass sie Aluminiumionen in einem Anteil im Bereich von 0,5 - 15 Gewichts-% (ggf. umgerechnet auf $Al^{3+}$) bezogen auf die Gesamtmasse der Lösung umfasst und weiterhin Anionen der Milchsäure (Laktat-Ionen) und der Phosphorsäure (Phosphat-Ionen) umfasst, wobei der molare Anteil der Laktat-Ionen im Bereich des 0,01 - 2,99-fachen bezogen auf den molaren Anteil an $Al^{3+}$ ist und der molare Anteil der Phosphat-Ionen im Bereich des 0,01 - 2,99-fachen bezogen auf den molaren Anteil an $Al^{3+}$ ist, umfasst.

Die erfindungsgemäßen Superabsorber zeigen überraschend gute Permeabilität und Stabilität gegen Verfärbung, ohne dass ihre übrigen Gebrauchseigenschaften wie CRC oder AUL wesentlich beeinträchtigt wären. Durch das zusätzlich zu Aluminiumionen und Phosphationen enthaltene Laktat kann eine stabile Lösung erhalten werden.

Zusätzlich zu Laktat und Phosphat können auch andere Anionen in der erfindungsgemäß zu verwendenden Lösung enthalten sein. Das Vorhandensein von Laktat scheint jedoch für die Stabilisierung der aluminium- und phosphathaltigen Lösung vorteilhaft oder gar zwingend notwendig zu sein. Eine laktatfreie, aber Aluminiumionen und Phosphationen enthaltende stabile Lösung scheint insbesondere aber auch in Form einer Aluminiumionen, Phosphationen und Sulfationen, enthaltenden Lösung zugänglich zu sein. Der Sulfatanteil verringert jedoch die Stabilität gegen Verfärbungen. Vorzugsweise wird daher als erfindungsgemäß zu verwendende Aluminiumionen enthaltende Lösung eine Lösung verwendet, die höchstens 5 Gew.-% Sulfationen, vorzugsweise höchstens 3 Gew.-% Sulfationen und in besonders bevorzugter Form höchstens 1 Gew.-% Sulfationen enthält. In ganz besonders bevorzugter Weise ist die Lösung sulfatfrei, das heißt, frei von absichtlich zugesetztem Sulfat.

Weiterhin wurden Artikel zur Absorption von Flüssigkeiten gefunden, insbesondere Hygieneartikel zur Absorption von flüssigen Körperausscheidungen oder flüssigen Anteilen von Körperausscheidungen, die dadurch gekennzeichnet sind, dass sie den erfindungsgemäßen Superabsorber enthalten. Ferner wurden Verfahren zur Herstellung solcher Artikel zur Absorption von Flüssigkeiten gefunden, die dadurch gekennzeichnet sind, dass man bei der Herstellung dieser Artikel ihnen den erfindungsgemäßen Superabsorber zusetzt.

Die Oberflächen des erfindungsgemäßen Superabsorbers sind mit Aluminiumionen komplexiert. Superabsorber werden ganz überwiegend in Form von Pulvern hergestellt, in den meisten Fällen bedeutet Komplexierung seiner Oberflächen also Komplexierung der Partikeloberflächen. Mache Superabsorber werden aber auch in anderen Formen erzeugt, beispielsweise als Schäume, Fasern, Rollgut oder als auf einem Träger, etwa einem Vlies, fixierte Superabsorberpartikel. Auch die Oberflächen solcher Superabsorber können mit Aluminium komplexiert sein. Die Komplexierung der Oberfläche von Superabsorbern ist an sich bekannt. "Komplexierung" ist streng genommen lediglich ein eigener Begriff für den Sonderfall der Oberflächennachvernetzung, bei dem durch Aluminiumionen ionische Bindungen zwischen mehreren polaren Gruppen an der Oberfläche der Superabsorberpartikel erzeugt werden. Oft wird die Komplexierung auch unter "Oberflächennachvernetzung" diskutiert. Im Rahmen dieser Erfindung wird unter "Komplexierung" die Oberflächennachvernetzung mit Aluminiumionen verstanden, um sie von der Oberflächennachvernetzung mit Nachvernetzern, die kovalente Bindungen mit polaren Gruppen an der Oberfläche der Superabsorberpartikel ausbilden, abzugrenzen.

Aluminiumionen werden dem Superabsorber bei der Komplexierung im Allgemeinen in einer Menge von mindestens 0,008 Gew.-%, vorzugsweise mindestens 0,015 Gew.-% und besonders bevorzugt mindestens 0,020 Gew.-% sowie im Allgemeinen von höchstens 0,15 Gew.-%, vorzugsweise höchstens 0,10 Gew.-% und besonders bevorzugt höchstens 0,05 Gew.-%, jeweils berechnet als Metall (oder $Al^{3+}$-Ion) und bezogen auf die Gesamtmenge des wasserfreien Superabsorbers zugesetzt.

In einer bevorzugten Ausführungsform kann die erfindungsgemäß verwendete wässrige Lösung weiterhin ein Anion mindestens einer dritten Säure enthalten, wobei die dritte Säure bevorzugt ausgewählt ist aus einer Gruppe, die Aminosäuren, Carbonsäuren, Citronensäure, Weinsäure, Äpfelsäure, Oxalsäure, Glykolsäure, Bernsteinsäure, Gluconsäure, Glycin, Essigsäure, Schwefelsäure und/oder Kombinationen umfasst. Nicht durch Laktat, Säureanion (der dritten Säure) und Phosphat ausgeglichene Ladungen können z.B. durch OH⁻-Ionen ausgeglichen werden.

In einer bevorzugten Ausführungsform kann neben Aluminium auch eine weitere Kationenart oder mehrere weitere Kationenarten in der Lösung enthalten sein. Die Menge und die Anzahl verschiedener dieser Kationen übersteigt dabei die als Verunreinigung - insbesondere beim Einsatz von Substanzen in technischer Qualität - ohnehin vorhandenen Kationen. Daher soll im Folgenden als weiteres Kation jeglicher gezielter bzw. gewünschter Zusatz von Kationen verstanden werden. Als besonders bevorzugte Kationen haben sich Alkali- und Erdalkali-Ionen erwiesen. Diese bieten sich insbesondere deshalb an, da sie meist leicht lösliche Salze bilden und daher einerseits leicht löslich sind, andererseits auch selten zum Ausfallen aus einer Al-haltigen Lösung neigen. Alternativ oder zusätzlich zu diesen Kationen sind auch Kationen der Übergangs- oder Seltenerdmetallen als kationische Zusätze möglich. In einer besonders bevorzugten Ausführungsform handelt es sich bei dem zugesetzten Kation um ein Ammonium-Ion. Alternativ oder ergänzend dazu haben sich Na⁺, K⁺, Ca²⁺, Mg²⁺ oder Zr⁴⁺ als kationische Zusätze in Al-Laktat-Phosphat-Lösungen erwiesen. In einer bevorzugten Ausführungsform zeichnet sich die wässrige Lösung dadurch aus, dass sie einen Zusatz eines Kations umfasst, wobei das Kation bevorzugt ausgewählt ist aus einer Gruppe, die Alkaliionen, Erdalkaliionen, Ammonium-Ionen, Kationen eines oder mehrerer Übergangs- oder Seltenerdmetalle oder Kombinationen davon umfasst. In einer besonders bevorzugten Aus- führungsform ist ein zugesetztes Kation ausgewählt aus der Gruppe, die Na⁺, K⁺, Ca²⁺, Mg²⁺, Zr⁴⁺, $NH_4^+$ oder Kombinationen davon umfasst.

In einer weiteren bevorzugten Ausführungsform weist die Lösung Cluster der theoretischen Zusammensetzung $Al^{3+}{}_A(C_3H_5O_3^-)_{x \cdot A}S^{M-}{}_{y \cdot A}(H_2PO_4^-)_{z \cdot A}(OH^-)_{(3A-x \cdot A-M \cdot y \cdot A-z \cdot A)}$ auf, wobei S das Anion einer optional vorhandenen dritten Säure mit der Ladung M ist, x ein Wert im Bereich von 0,01 - 2,99, bevorzugt 0,5 - 2,8, weiter bevorzugt 0,75 - 2,0, meist bevorzugt 1,0 - 1,5 ist, y ein Wert im Bereich von 0 - 2,8, bevorzugt 0 - 2, bevorzugt 0 - 1,25, besonders bevorzugt 0 - 1,0 ist und z ein Wert im Bereich von 0,05 - 2,9, bevorzugt, 0,1 - 2,5, weiter bevorzugt 0,2 - 1,5, besonders bevorzugt 0,3 - 1,25 ist. Die Summe aus x, M•y und z ist bevorzugt ≤ 3, so dass gemäß der obigen Formel die positive Ladung der Aluminiumionen ausgeglichen wird. Jedoch sind auch andere (unten näher beschriebene andere) Kationen in der Lösung denkbar, so dass die Summe aus x, M•y und z ist auch größer als 3 sein kann. Da Laktat und Phosphat zwingend vorhanden sind, um eine stabile aluminium- und phosphathaltige Lösung erhalten zu können, sind x und z immer > 0.

Laktationen sind dabei bevorzugt, aber nicht zwingend erforderlich die - bezogen auf den molaren Anteil der gelösten Stoffe - am häufigsten vorkommende anionische Komponente in der Lösung. Wie aus obiger Formel ersichtlich ist, kann x in den Clustern einen Wert im Bereich von 0,5 - 2.8 annehmen, so dass bei einem Faktor von 2,8 bereits ein Großteil der durch die Aluminiumionen in die Lösung eingebrachten Ladung durch Laktationen kompensiert ist. Der Anteil an Phosphationen ist somit dann entsprechend klein. Weitere durch Aluminiumkationen eingebrachte Ladungen können durch die zusätzliche Säure S oder die optional vorhandenen OH⁻-Ionen kompensiert werden.

In einer bevorzugten Ausführungsform ist Y > 0. Somit ist gemäß der oben genannten Formel mindestens ein Anion einer dritten Säure enthalten. Bevorzugt ist diese Säure bzw. dessen Anion ausgewählt aus einer Gruppe, die Aminosäuren, Carbonsäuren, Citrat, Tartrat, Malat, Oxalat, Glykolat, Succinat, Gluconat, Glycinat, Acetat, Sulfat und/oder Kombinationen davon umfasst. Untersuchungen haben gezeigt, dass durch diese Anionen die Stabilität der wässrigen Lösung weiter gesteigert werden kann. Außerdem ist in einigen Fällen eine Steigerung der Konzentration an Aluminiumionen und/oder Phosphationen möglich.

In einer bevorzugten Ausführungsform zeichnet sich die wässrige Lösung dadurch aus, dass sie in einem Temperaturbereich von 0 - 80°C stabil ist. Besonders bevorzugt sind wässrige Lösungen, die auch bei niedrigen Temperaturen lagerstabil sind. Bevorzugt sind Lösungen, die bei <30°C, bevorzugt <20°C, besonders bevorzugt <10°C lagerstabil sind. Als lagerstabil sollen Zeiträume verstanden werden, die bevorzugt 1 Monat, bevorzugt 6 Monate, besonders bevorzugt 12 Monate übersteigen. Bevorzugt ist somit eine wässrige Lösung länger als 1 Monat, bevorzugt länger als 6 Monate, besonders bevorzugt länger als 12 Monate lagerstabil. Diese Lagerstabilität erlaubt es, diese Lösungen bis zu deren Einsatz vorzuhalten und/oder zu transportieren. Auch der Transport über lange Strecken, z. B. per LKW oder auch per Schiff ist bei solcher Lagerstabilität möglich.

Um den Phosphatgehalt der Lösung möglichst hoch zu halten, ist bevorzugt, dass ein möglichst hoher Anteil der Anionen Phosphationen sind. Gemäß der obigen Formel ist x somit bevorzugt vergleichsweise klein. In einer bevorzugten Ausführungsform der vorliegenden Erfindung ist x in einem Bereich von 0,5 - 2,5, bevorzugt im Bereich von 0,75 - 2,0, besonders bevorzugt im Bereich zwischen 1,0 und 1,5. Im Bereich von x = 1,0 bis x = 1,5 wird somit ein Drittel bis die Hälfte der durch die dreifach positiv geladenen Aluminiumkationen in die Lösung eingebrachten positiven Ladungen durch Laktat kompensiert. Die übrige zur Kompensation der positiven Ladung notwendige negative Ladung kann durch Anionen S der Säure, Phosphationen oder OH⁻-Ionen bereitgestellt werden. Die Stabilität der Lösung nimmt bei einem Wert von x < 0,5 ab. Es scheint, dass bei geringen Laktatanteilen die Stabilisierung der Lösung nicht ausreichend ist und es daher in Abhängigkeit von den Lagerbedingungen zum Ausfallen von Aluminiumphosphat kommen kann.

Auch für die Menge an Anionen der zusätzlichen dritten Säure (S) gilt, dass diese möglichst klein sein sollte, um den Phosphatgehalt möglichst groß halten zu können. In einer bevorzugten Ausführungsform ist Y daher kleiner als 1,25, bevorzugt kleiner als 1,0, besonders bevorzugt kleiner als 0,8.

In einer bevorzugten Ausführungsform weist die dritte Säure eine zusätzliche Funktionalität auf. Eine solche zusätzliche Funktionalität ist besonders bevorzugt ausgewählt aus einer Gruppe, die Substituenten umfasst, die eine Aminofunktion, eine weitere Säurefunktion, eine Carbonylfunktion, eine Doppelbindung, eine Dreifachbindung, ein Heteroatom, eine Ladung, eine Partialladung und/oder Kombinationen davon in das Molekül einbringen. Insbesondere dann, wenn die Funktionalität der dritten Säure dazu geeignet ist, die Eigenschaften der gewünschten Lösung positiv zu verändern, kann der Anteil der dritten Säure sehr hoch sein und beispielsweise den Anteil an Phosphorsäure und/oder Milchsäure sogar übersteigen. Insbesondere wenn die Einstellung eines bestimmten pH-Wertes gewünscht ist, kann dies in einigen bevorzugten Ausführungsformen durch die Auswahl einer geeigneten dritten Säure, welche entsprechende Funktionalitäten mitbringt, realisiert sein. So könnte beispielsweise auch eine Puffer-Funktion z.B. durch eine Aminofunktion und/oder eine (weitere) Säurefunktion realisiert werden. Somit wäre es möglich, den pH-Wert auch bei Veränderung der Umgebungsbedingungen (z.B. Temperaturschwankungen, Verunreinigungen, Verdünnungen (z.B. durch Regenwasser)) zumindest weitgehend konstant zu halten und das Ausfallen von Salzen und/oder anderen Feststoffen zu vermeiden.

Es hat sich gezeigt, dass es insbesondere für die Langzeitlagerstabilität vorteilhaft ist, wenn X>Z ist. In einer bevorzugten Ausführungsform ist somit der Anteil an Laktationen in der Lösung größer als der der Phosphationen. Dies ist jedoch wie unten gezeigt nicht zwingend erforderlich.

Um die Langzeitstabilität der Lösung zu gewährleisten ist bevorzugt, dass die Konzentration der Aluminiumionen im Bereich von 1-10 Gewichts-% (ggf. umgerechnet auf $Al^{3+}$) bezogen auf die Gesamtmasse der Lösung liegt. Weiter bevorzugt liegt die Konzentration der Aluminiumionen (ggf. umgerechnet auf $Al^{3+}$) im Bereich von 1,5 - 5 Gewichts-%.

In einer weiteren bevorzugten Ausführungsform zeichnet sich die wässrige Lösung dadurch aus, dass in der theoretischen Zusammensetzung $Al^{3+}_A(C_3H_5O_3^-)_{x \cdot A}S^{M-}_{y \cdot A}(H_2PO_4^-)_{z \cdot A}(OH^-)_{(3A-x \cdot A-M \cdot y \cdot A-z \cdot A)}$ der gelösten Stoffe der Index $(3A-x \cdot A-M \cdot y \cdot A-z \cdot A) > 0$ ist. Somit liegen bevorzugt $OH^-$-Ionen vor. Besonders bevorzugt ist, dass der Index $(3A-x \cdot A-M \cdot y \cdot A-z \cdot A)$ im Bereich von 0,5 bis etwa 1,75 liegt (siehe Beispiele unten). Die Summe aus x, $M \cdot y$ und z liegt somit bevorzugt im Bereich 1,25 bis 2,5.

Der Lösung können weitere Stabilisatoren (wie z. B. Chelatbilder, Polymere) zugesetzt werden, beispielsweise der Zusatz von einer Substanz oder mehrerer Substanzen, die unabhängig voneinander ausgewählt ist/sind aus einer Gruppe, die Acetylaceton (acac), Ethylendiamin (en), 2-(2-Aminoethylamino)ethanol (AEEA), Diethylentriamin (dien), Iminodi- acetat (ida), Triethylentetramin (trien, TETA), Triaminotriethylamin (tren), Nitrilotriacetat (nta), Bis(salicyliden)ethylendiamin (salen), Ethylendiaminotriacetat (ted), Ethylendiamintetraacetat (EDTA), Diethylentriaminpentaacetat (DTPA), 1,4,7,10-Tetraazacyclododecan-1,4,7,10-tetra-acetat (DOTA), Dimethylglyoxim (dmg), 8-Hydroxychinolin (oxin), 2,2'-Bipyridin (bpy), 1,10- Phenanthrolin (phen), Dimercaptobernsteinsäure (DMSA), 1,2-Bis(diphenylphosphino)ethan (dppe) oder andere umfasst. Insbesondere sind Chelatbilder bevorzugt, bei denen mindestens eine energetisch positive Wechselwirkung zwischen dem Chelatbilder und dem zu komplexierenden Kation über ein Heteroatom ausgebildet wird, welches ausgewählt ist aus einer Gruppe, die N, P und S umfasst, oder eine funktionelle Gruppe, die ein Sauerstoffatom umfasst und ausgewählt ist aus einer Gruppe, die Ketone, (A(C=O)B mit A, B = organischer Rest), Aldehyde (A = H, B = organischer Rest oder H), Ester (A = O-R, B = organischer Rest oder H), Amide (A = $NH_2$, NHR, $NR^1R^2$, B = organischer Rest oder H), Harnstoffe (A, B = $NH_2$, NHR, $NR^1R^2$), Urethane (A = OR, B = $NH_2$, NHR, $NR^1R^2$), Alkohole (A-OH, A = organischer Rest) umfasst. Es ist jedoch bevorzugt, dass die Lösung keine weiteren Stabilisatoren enthält. Insbesondere ist bevorzugt, dass die Lösung keine der zuvor genannten Stabilisatoren und insbesondere keine der oben genannten Chelatbildner umfasst.

Hergestellt werden die erfindungsgemäß zu verwendenden Aluminiumlösungen beispielsweise durch ein Verfahren, das sich durch die Schritte

   a) Bereitstellen von Wasser in einem Reaktionsgefäß,
   b) Zugabe eines basischen Aluminiumsalzes, welches bevorzugt ausgewählt ist aus einer Gruppe, die Al-Carbonat, Al-Hydroxid, Al-Oxid, ein Aluminat oder Kombinationen davon enthält, unter Rühren in das Reaktionsgefäß,
   c) Zugabe von Milchsäure und/oder eines Laktats und parallel dazu oder zeitlich versetzt davon von Phosphorsäure und/oder eines Phosphats,
   d) Rühren der erhaltenen Mischung

auszeichnet, wobei die Reihenfolge der Ausführung der Schritte b) und c) beliebig ist.

Es konnte gezeigt werden, dass bei der oben genannten Abfolge der einzelnen Prozessschritte eine stabile Lösung erhalten werden kann. Dieses Verfahren ermöglicht die Herstellung großer Mengen einer gewünschten, bevorzugt wie oben beschriebenen Lösung. Beispielsweise können so in einem Ansatz mehr als 1 $m^3$ der oben beschriebenen Lösung hergestellt werden.

In einer spezifischen Ausführungsform der genannten Abfolge a) bis d) wird zur Herstellung einer solchen Lösung Aluminium in Form eines basischen Salzes (Carbonat, Hydroxid, Oxid, Aluminat, bevorzugt Hydroxid) in wässriger Suspension mit 10 - 20 Gewichts-% Feststoff vorgelegt. Ein geeigneter Einsatzstoff sind etwa die amorphen sogenannten "Aluminiumoxidhydrate", die in bekannter Weise beim Fällen aus Aluminiumsalzlösungen durch Basenzugabe entstehen. Die Verwendung kristalliner Aluminiumhydroxide wie etwa Bayerit (alpha-Al(OH)$_3$), Hydrargillit (gamma-Al(OH)$_3$), Böhmit (alpha-AlO(OH)) oder Diaspor (gamma-AlO(OH)) ist zwar nicht ausgeschlossen, ist aber weniger bevorzugt, weil diese Aluminiumsalze sich typischerweise langsamer in Säuren lösen als amorphes Aluminiumhydroxid oder -oxidhydrat. Am reaktivsten ist naturgemäß frisch gefälltes Aluminiumhydroxid oder -oxidhydrat, aber auch die handelsüblichen Aluminiumhydroxide oder -oxidhydrate sind gut geeignet. Ein Beispiel eines geeigneten Rohstoffs ist Aluminiumoxid, hydratisiert (Pulver, gemäß Ph. Eur. 9. Ed., 47,0-60,0 % Al$_2$O$_3$, Dr. Paul Lohmann GmbH KG, Artikelnummer 511066100). Anschließend werden unter Rühren Milchsäure und Phosphorsäure hinzugegeben und bei einer Temperatur von etwas über 40°C zur Reaktion gebracht. Dabei entsteht eine klare bis leicht trübe Lösung, die mittels Filtration von eventuell enthaltenen (ungelösten oder ausgefallenen) Feststoffanteilen befreit wird.

Ist das Vorhandensein einer weiteren anionischen Komponente vorgesehen, so wird diese bevorzugt in Form einer Säure oder eines leicht löslichen Salzes zugegeben. Weiter bevorzugt erfolgt die Zugabe zusammen mit den übrigen in Schritt c) genannten Substanzen oder während oder nach Schritt d).

In einer bevorzugten Variante des Verfahrens ist vorgesehen, dass Cluster der theoretischen Zusammensetzung Al$^{3+}_A$(C$_3$H$_5$O$_3^-$)$_{x \cdot A}$S$^M{}_{y \cdot A}$(H$_2$PO$_4^-$)$_{z \cdot A}$(OH$^-$)$_{(3A \cdot x \cdot A-M \cdot y \cdot A-z \cdot A)}$ erzeugt werden, wobei S das Anion einer optional vorhandenen dritten Säure mit der Ladung M ist, x ein Wert im Bereich von 0,01 - 2,99, bevorzugt 0,5 - 2,8, weiter bevorzugt 0,75 - 2,0, meist bevorzugt 1,0 - 1,5 ist, y ein Wert im Bereich von 0 - 2,8, bevorzugt 0 2, bevorzugt 0 - 1,25, besonders bevorzugt 0 - 1,0 ist und z ein Wert im Bereich von 0,05 - 2,9, bevorzugt, 0,1 - 2,5, weiter bevorzugt 0,2 - 1,5, besonders bevorzugt 0,3 - 1,25 ist.

Insbesondere ist verfahrensseitig bevorzugt, dass Schritt c) bei einer Temperatur >25°C, bevorzugt >40°C, weiter bevorzugt >50°C, besonders bevorzugt bei 60 - 70°C oder alternativ dazu unter Rückfluss erfolgt. Bei diesen Temperaturen wird der Mischung bzw. Lösung genügend Energie zugeführt, um ein Ausfällen von Aluminiumphosphat weitgehend zu verhindern. Es wird vermutet, dass bei diesen Temperaturen genügend thermische Energie zur Verfügung steht, so dass die Ionen Cluster ausbilden können. Diese Cluster sind löslich und/oder bleiben kolloidal in Lösung und fallen auch bei anschließendem Unterschreiten der oben genannten Temperatur nicht aus.

Bei einigen Zusammensetzungen und/oder Varianten des Verfahrens ist es nicht vollständig ausgeschlossen, dass ein geringer Anteil des eingesetzten Aluminiums und/oder Phosphats z. B. als Aluminiumphosphat oder auch zusammen mit anderen Substanzen ausfällt. Auch Verunreinigungen der Ausgangsmaterialien können als unlösliche Bestandteile in der Lösung bzw. Mischung enthalten sein. In diesem Fall ist eine Filtration vorteilhaft. In einer bevorzugten Verfahrensvariante zeichnet sich das Verfahren daher dadurch aus, dass auf Schritt c) eine Filtration folgt. Bevorzugt wird diese durch eine Filtration in einer Filterpresse durchgeführt. Als Produkt dieses Filtrationsschrittes erhält man eine klare Lösung, die den Anforderungen in Bezug auf die gewünschte Stabilität und den Gehalt an Aluminium- und Phosphationen genügt.

Die so hergestellte Lösung wird zur Komplexierung des Superabsorbers mit Aluminiumionen eingesetzt.

Der erfindungsgemäße Superabsorber ist vorzugsweise zusätzlich zur Komplexierung auch mit Nachvernetzern, die kovalente Bindungen mit polaren Gruppen an der Oberfläche der Superabsorberpartikel ausbilden, an seiner Oberfläche nachvernetzt.

Die erfindungsgemäßen Superabsorber sind beispielsweise mit dem erfindungsgemäßen Verfahren erhältlich. Es ist jedoch ebenso möglich, auch nicht durch die beschriebene Lösungspolymerisation erhaltene Superabsorber mit der erfindungsgemäß zu verwendenden Aluminiumlösung zu komplexieren.

Das erfindungsgemäße Verfahren zur Herstellung von Superabsorbern ist ein Verfahren zur wässrigen Lösungspolymerisation einer Monomermischung, die Folgendes umfasst:

a) mindestens ein ethylenisch ungesättigtes, mindestens eine Säuregruppe tragendes Monomer, das wahlweise zumindest teilweise als Salz vorliegt,
b) mindestens einen Vernetzer,
c) mindestens einen Initiator,
d) wahlweise ein oder mehrere mit den unter a) genannten Monomeren copolymerisierbare ethylenisch ungesättigte Monomere und
e) wahlweise ein oder mehrere wasserlösliche Polymere.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren oder ihre Salze, wie Acrylsäure, Methacrylsäure, Maleinsäure oder ihre Salze, Maleinsäureanhydrid und Itaconsäure oder ihre Salze. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomerlösung enthält vorzugsweise höchstens 250 Gew.-ppm, bevorzugt höchstens 130 Gew.-ppm, besonders bevorzugt höchstens 70 Gew.-ppm sowie bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a), wobei neutralisiertes Monomer a), d.h. ein Salz des Monomers a) rechnerisch als unneutralisiertes Monomer berücksichtigt wird. Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 530 438 A1 beschrieben, Di- und Triacrylate, wie in EP 547 847 A1, EP 559 476 A1, EP 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/32962 A2 beschrieben.

Bevorzugte Vernetzer b) sind Pentaerythrittriallylether, Tetraallyloxyethan, Methylenbismethacrylamid, 15-bis 20-fach ethoxiliertes Trimethylolpropantriacrylat, 15-20-fach ethoxiliertes Glycerintriacrylat, Polyethylenglykoldiacrylat mit zwischen 4 und 45 -CH$_2$CH$_2$O-Einheiten in der Molekülkette, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,3 bis 0,6 Gew.-%, jeweils bezogen auf Monomer a). Mit steigendem Vernetzergehalt sinkt die Zentrifugenretentionskapazität (CRC) und steigt die Absorption unter Druck (AUL).

Als Initiatoren c) können sämtliche unter den Polymerisationsbedingungen in Radikale erzeugende Verbindungen eingesetzt werden, beispielsweise thermische Initiatoren, Redox-Initiatoren und/oder Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise auch ein Sulfonsäurederivat eingesetzt, beispielsweise ein Gemischen aus dem Natriumsalz der 2-Hydroxi-2-sulfinatoessigsäure, dem Dinatriumsalz der 2-Hydroxi-2-sulfonatoessigsäure und Natriumbisulfit, das beispielsweise von L. Brüggemann KG (Salzstraße 131, 74076 Heilbronn, Deutschland, www.brueggemann.com) unter den Bezeichnungen BRÜGGOLIT® FF6M oder BRÜGGOLIT® FF7, alternativ BRUGGO-LITE® FF6M oder BRUGGOLITE® FF7 erhältlich ist. oder das Dinatriumsalz der 2-Hydroxi-2-sulfonatoessigsäure, das beispielsweise von L. Brüggemann KG unter der Bezeichnung BLAN-COLEN® HP erhältlich ist. Die Initiatoren werden im Übrigen in üblichen Mengen verwendet. Die übliche Menge der reduzierenden Komponente eines Redoxinitiators liegt im Allgemeinen bei mindestens 0,00001 Gew.-%, vorzugsweise mindestens 0,0001 Gew.-% und besonders bevorzugt mindestens 0,001 Gew.-% sowie im Allgemeinen von höchstens 0,2 Gew.-% und vorzugsweise höchstens 0,1 Gew.-%, jeweils bezogen auf die Menge der Monomeren a) und d). Wird als reduzierende Komponente des Redoxinitiators jedoch allein Sulfonsäurederivat eingesetzt, liegt dessen zugesetzte Menge im Allgemeinen bei mindestens 0,001 Gew.-%, vorzugsweise mindestens 0,01 Gew.-% und besonders bevorzugt mindestens 0,03 Gew.-% sowie im Allgemeinen von höchstens 1,0 Gew.-%, vorzugsweise höchstens 0,3 Gew.-%, und besonders bevorzugt bei höchstens 0,2 Gew.-%, jeweils bezogen auf die Menge der Monomeren a) und d). Die übliche Menge der oxidierenden Komponente eines Redoxinitiators liegt im Allgemeinen bei 0,0001 Gew,-% und besonders bevorzugt mindestens 0,001 Gew.-% sowie im Allgemeinen von höchsten 2 Gew.-% und vorzugsweise höchstens 1,0 Gew.-%, jeweils bezogen auf die Menge der Monomeren a) und d). Die übliche Menge der thermischen Initiatoren liegt im Allgemeinen bei 0,01 Gew,-% und besonders bevorzugt mindestens 0,1 Gew.-% sowie im Allgemeinen von höchsten 2 Gew.-% und vorzugsweise höchstens 1,0 Gew.-%, jeweils bezogen auf die Menge der Monomeren a) und d). Die übliche Menge der Photoinitiatoren liegt im Allgemeinen bei 0,001 Gew,-% und besonders bevorzugt mindestens 0,01 Gew.-% sowie im Allgemeinen von höchsten 1,0 Gew.-% und vorzugsweise höchstens 0,2 Gew.-%, jeweils bezogen auf die Menge der Monomeren a) und d).

Mit den ethylenisch ungesättigten, säuregruppentragenden Monomeren a) copolymerisierbare ethylenisch ungesättigte Monomere d) sind beispielsweise Acrylamid, Methacrylamid, Hydroxiethylacrylat, Hydroxiethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylacrylat, Maleinsäure oder ihre Salze und Maleinsäureanhydrid.

Als wasserlösliche Polymere e) können Polyvinylalkohol, Polyvinylpyrrolidon, Stärke, Stärkederivate, modifizierte Cellulose, wie Methylcellulose oder Hydroxiethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. übersättigte Monomerlösungen einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Die Monomermischung kann weitere Komponenten enthalten. Beispiele in derartigen Monomermischungen verwendeter weiterer Komponenten sind etwa Chelatbildner, um Metallionen in Lösung zu halten oder anorganische Pulver, um die Steifigkeit des Superabsorbers im gequollenen Zustand zu erhöhen, oder rückgeführtes Unterkorn aus einer späteren Mahlung. Hier können alle bekannten Zusätze zur Monomermischung verwendet werden. Auch wenn im Zusammenhang mit der Monomermischung hier nur von "Lösung" die Rede ist, ist hiermit auch die Polymerisation einer Suspension mit gemeint, etwa wenn der Monomermischung unlösliche Bestandteile zugesetzt werden.

Die Säuregruppen der aus der Polymerisation erhaltenen Polymergele sind üblicherweise teilweise neutralisiert. Die Neutralisation wird vorzugsweise auf der Stufe der Monomeren durchgeführt, mit anderen Worten, es werden Salze der säuregruppentragenden Monomeren oder genaugenommen eine Mischung von säuregruppentragenden Monomeren und Salzen der säuregruppentragenden Monomeren ("teilneutralisierte Säure") als Komponente a) in die Polymerisation eingesetzt. Dies geschieht üblicherweise durch Einmischung des Neutralisationsmittels als wässrige Lösung oder bevorzugt auch als Feststoff in die zur Polymerisation vorgesehene Monomermischung oder bevorzugt in das säuregruppentragende Monomer oder eine Lösung davon. Der Neutralisationsgrad beträgt vorzugsweise von 25 bis 95 mol-%, besonders bevorzugt von 50 bis 80 mol-%, ganz besonders bevorzugt von 65 bis 72 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Es ist jedoch bevorzugt, die Neutralisation auf der Stufe des Monomeren durchzuführen. Mit anderen Worten: in einer ganz besonders bevorzugten Ausführungsform wird als Monomer a) ein Gemisch aus 25 bis 95 mol-%, besonders bevorzugt von 50 bis 80 mol-%, ganz besonders bevorzugt von 65 bis 75 mol-% Salz des säuregruppentragenden Monomeren und dem Rest zu 100 mol-% säuregruppentragendes Monomer eingesetzt. Dieses Gemisch ist beispielsweise ein Gemisch aus Natriumacrylat und Acrylsäure oder ein Gemisch aus Kaliumacrylat und Acrylsäure.

In einer bevorzugten Ausführungsform wird zur Neutralisation ein Neutralisationsmittel verwendet, dessen Gehalt an Eisen im Allgemeinen unter 10 Gew.-ppm, vorzugsweise unter 2 Gew.-ppm und in besonders bevorzugter Weise unter 1 Gew.-ppm liegt. Ebenso ist ein niedriger Gehalt an Chlorid sowie Anionen von Sauerstoffsäuren des Chlors erwünscht. Ein geeignetes Neutralisationsmittel ist beispielsweise die üblicherweise als "membrane grade" gehandelte 50 Gew.-%ige Natronlauge oder Kalilauge, noch reiner und ebenso geeignet, allerdings auch kostspieliger ist die üblicherweise als "amalgame grade" oder "mercury process" gehandelte 50 Gew.-%ige Natronlauge oder Kalilauge.

Verfahren zur Herstellung von Superabsorbern aus Monomermischungen wie der vorstehend beispielhaft beschriebenen sind grundsätzlich bekannt. Geeignete Polymerisationsreaktoren sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/38402 A1 beschrieben. Die Polymerisation auf dem Band wird beispielsweise in EP 955 086 A2, DE 38 25 366 A1 und US 6,241,928 beschrieben. Bei der Polymerisation in einem Bandreaktor entsteht wie auch bei der ebenso bekannten Polymerisation im Satzbetrieb oder im Rohrreaktor, wie beispielsweise in EP 445 619 A2 and DE 19 846 413 A1 beschrieben, ein Polymergel, das in einem weiteren Verfahrensschritt zerkleinert werden muss, beispielsweise in einem Fleischwolf, Extruder oder Kneter. Es können aber auch sphärische oder anders geformte Superabsorberpartikel durch Suspensions- oder Emulsionspolymerisation hergestellt werden, wie beispielsweise in EP 457 660 A1 beschrieben, oder durch Sprüh- oder Tropfenpolymerisationsverfahren hergestellt werden, wie beispielsweise in EP 348 180 A1, EP 816 383 A1, WO 96/404 27 A1, US 4 020 256, US 2002/0 193 546 A1, DE 35 19 013 A1, DE 10 2005 044 035 A1, WO 2007/093531 A1, WO 2008/086 976 A1 oder WO 2009/027 356 A1 beschrieben. Ebenso bekannt sind Verfahren, bei denen die Monomermischung auf ein Substrat wie beispielsweise eine Vliesbahn aufgebracht und polymerisiert wird, wie etwa in WO 02/94 328 A2 und WO 02/94 329 A1 beschrieben.

Dem Superabsorber oder auch der Monomermischung kann in bekannter Weise vor oder nach der Trocknung, vorzugsweise aber vor der Trocknung, wahlweise ein Sulfonsäurederivat zugesetzt werden, auch im Gemisch mit Sulfit oder Sulfinsäurederivat. Diese Gemische sind gängige Handelswaren und beispielsweise in der Form von Gemischen aus dem Natriumsalz der 2-Hydroxi-2-sulfinatoessigsäure, dem Dinatriumsalz der 2-Hydroxi-2-sulfonatoessigsäure und Natriumbisulfit von L. Brüggemann KG (Salzstraße 131, 74076 Heilbronn, Deutschland, www.brueg-gemann.com) unter den Bezeichnungen BRÜGGOLIT® FF6M oder BRÜGGOLIT® FF7, alternativ BRUGGOLITE® FF6M oder BRUGGO-LITE® FF7 erhältlich. Bevorzugt ist die Verwendung der Sulfonsäurederivate in Reinform. Auch diese sind gängige Handelswaren. So ist beispielsweise das Dinatriumsalz der 2-Hydroxi-2-sulfonatoessigsäure von L. Brüggemann KG (Salzstraße 131, 74076 Heilbronn, Deutschland, www.brueggemann.com) unter der Bezeichnung BLANCOLEN® HP erhältlich.

Das Sulfonsäurederivat wird im Allgemeinen in einer Menge von mindestens 0,0001 Gew.-%, vorzugsweise mindestens 0,001 Gew.-% und in besonders bevorzugter Form mindestens 0,025 Gew.-%, beispielsweise mindestens 0,05 Gew.-% oder mindestens 0,1 Gew.-% sowie im Allgemeinen höchstens 3 Gew.-%, in bevorzugter Form höchstens 2 Gew.-% und in besonders bevorzugter Form höchstens 0,5 Gew.-%, beispielsweise höchstens 0,35 Gew.-% oder 0,2 Gew.-%, jeweils auf das Gesamtgewicht des Superabsorbers bezogen, eingesetzt.

Dem Superabsorber oder auch der Monomermischung kann in bekannter Weise vor oder nach der Trocknung, vorzugsweise aber vor der Trocknung, ebenso wie das Sulfonsäurederivat zusätzlich zu diesem oder alleine wahlweise auch mindestens ein Phosphonsäurederivat zugesetzt werden. Besonders bevorzugt ist die Zugabe von bevorzugte Weise (1-Hydroxiethan-1,1-diyl)bisphosphonsäure ("Etidronsäure") oder eines Salzes davon, insbesondere dem Natriumsalz, dem Kaliumsalz, dem Dinatriumsalz, dem Dikaliumsalz oder dem Natrium-Kalium-Salz. Derartige Phosphonsäurederivate sind gängige Handelswaren und beispielsweise unter der Marke Cublen® von Zschimmer & Schwarz Mohsdorf GmbH & Co KG, Chemnitztalstraße 1, 09217 Burgstädt, Deutschland erhältlich.

Das Phosphonsäurederivat wird im Allgemeinen in einer Menge von mindestens 0,01 Gew.-%, vorzugsweise mindestens 0,1 Gew.-% und besonders bevorzugt mindestens 0,2 Gew.-% sowie im Allgemeinen von höchstens 1,9 Gew.-%, vorzugsweise höchstens 1,3 Gew.-% und besonders bevorzugt höchstens 0,6 Gew.-%, jeweils bezogen auf die Gesamtmenge des wasserfreien Superabsorbers zugesetzt.

Das aus der wässrigen Lösungspolymerisation und gegebenenfalls nachträglicher Neutralisation erhaltene Polymergel wird dann vorzugsweise mit einem Bandtrockner getrocknet, bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt (Messmethode für den Restfeuchte- oder Wassergehalt siehe unten). Bei einer zu hohen Restfeuchte weist das getrocknete Polymergel eine zu niedrige Glasübergangstemperatur Tg auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung im Allgemeinen von 25 bis 90 Gew.-%, vorzugsweise von 30 bis 80 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein beheizbarer Mischer mit mechanischem Mischorgan wie beispielsweise ein Schaufeltrockner oder ein ähnlicher Trockner mit anders gestalteten Mischwerkzeugen verwendet werden. Wahlweise kann der Trockner unter Stickstoff oder einem anderen nicht-oxidierenden Inertgas oder zumindest unter verringertem Partialdruck des Sauerstoffs betrieben werden, um oxidative Vergilbungsvorgänge zu verhindern. Im Regelfall führt aber auch eine ausreichende Belüftung und Abführung des Wasserdampfes zu einem akzeptablen Produkt. Vorteilhaft hinsichtlich Farbe und Produktqualität ist in der Regel eine möglichst kurze Trocknungszeit.

Während der Trocknung verringert sich auch der Restmonomerengehalt in den Polymerpartikeln und letzte Reste des Initiators werden zerstört.

Das getrocknete Polymergel wird hiernach wahlweise - und bevorzugterweise - gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen eingesetzt werden können. Übergroße, oft im Inneren noch nicht getrocknete Gelklumpen sind gummielastisch, führen zu Problemen bei der Mahlung und werden vorzugsweise vor der Mahlung abgetrennt, was durch Windsichtung oder ein Sieb ("Schutzsieb" für die Mühle) in einfacher Weise erfolgen kann. Die Maschenweite des Siebs ist angesichts der verwendeten Mühle so zu wählen, dass möglichst keine Störungen durch übergroße, gummielastische Partikel auftreten.

Zu große, nicht ausreichend fein gemahlene Superabsorberpartikel sind bei ihrer überwiegenden Verwendung, in Hygieneprodukten wie Windeln, als grobe Partikel fühlbar, sie senken auch die mittlere Anquellgeschwindigkeit des Superabsorbers Beides ist unerwünscht. Vorteilhafterweise werden daher grobkörnige Polymerpartikel aus dem Produkt abgetrennt. Dies erfolgt durch übliche Klassierverfahren, beispielsweise Windsichtung oder durch Siebung durch ein Sieb mit einer Maschenweite von höchstens 1000 $\mu$m, vorzugsweise höchstens 900 $\mu$m, besonders bevorzugt höchstens 850 $\mu$m und ganz besonders bevorzugt höchstens 800 $\mu$m. Beispielsweise werden Siebe mit 700 $\mu$m, 650 $\mu$m oder 600 $\mu$m Maschenweite verwendet. Die abgetrennten grobkörnigen Polymerpartikel ("Überkorn") können zur Kostenoptimierung dem Mahl- und Siebkreislauf wieder zugeführt oder separat weiterverarbeitet werden.

Polymerpartikel mit zu niedriger Partikelgröße senken die Permeabilität (SFC). Vorteilhafterweise werden daher bei dieser Klassierung auch feinkörnige Polymerpartikel abgetrennt. Dies kann, falls gesiebt wird, bequem durch ein Sieb mit einer Maschenweite von höchstens 300 $\mu$m, vorzugsweise höchstens 200 $\mu$m, in besonders bevorzugter Weise höchstens 150 $\mu$m und in ganz besonders bevorzugter Weise höchstens 100 $\mu$m erfolgen. Die abgetrennten feinkörnigen Polymerpartikel ("Unterkorn" oder "fines") können zur Kostenoptimierung beliebig dem Monomerstrom, dem polymerisierenden Gel, oder dem auspolymerisierten Gel vor der Trocknung des Gels wieder zugeführt werden.

Die mittlere Partikelgröße der als Produktfraktion abgetrennten Polymerpartikel beträgt im Allgemeinen mindestens 200 $\mu$m, bevorzugt mindestens 250 $\mu$m und in bevorzugter Form mindestens 300 $\mu$m sowie im Allgemeinen höchstens 600 $\mu$m und in bevorzugter Weise höchstens 500 $\mu$m. Der Anteil an Partikeln mit einer Partikelgröße von mindestens 150 $\mu$m beträgt im Allgemeinen mindestens 90 Gew.-%, bevorzugterweise mindestens 95 Gew.-% und in besonders bevorzugter Weise mindestens 98 Gew.-%. Der Anteil an Partikeln mit einer Partikelgröße von höchstens 850 $\mu$m, beträgt im Allgemeinen mindestens 90 Gew.-%, bevorzugterweise mindestens 95 Gew.-% und in besonders bevorzugter Weise mindestens 98 Gew.-%.

Bei manchen anderen bekannten Herstellverfahren für Superabsorber, insbesondere bei Suspensionspolymerisation, Sprüh- oder Vertropfungspolymerisation wird durch die Wahl der Verfahrensparameter die Partikelgrößenverteilung vorgegeben. Bei diesen Verfahren entsteht direkt partikulärer Superabsorber der gewünschten Partikelgröße, so dass Mahl- und Siebschritte oft entfallen können. In manchen Verfahren (insbesondere bei Sprüh- oder Vertropfungspolymerisation) kann oft auch ein eigener Trocknungsschritt entfallen.

Das so hergestellte Polymer hat superabsorbierende Eigenschaften und fällt unter den Begriff "Superabsorber". Seine CRC ist typischerweise vergleichsweise hoch, seine AUL oder SFC dagegen vergleichsweise niedrig. Ein derartiger, nicht oberflächennachvernetzter Superabsorber wird zur Unterscheidung von einem daraus hergestellten oberflächennachvernetzten Superabsorber oft "Grundpolymer" oder "Basispolymer" genannt.

Das Grundpolymer wird wahlweise oberflächennachvernetzt. Oberflächennachvernetzer für Superabsorber und Verfahren zur Oberflächennachvernetzung von Superabsorbern sind wohlbekannt. Geeignete Nachvernetzer sind Verbindungen, die Gruppen enthalten, die mit mindestens zwei funktionellen Gruppen der Superabsorberpartikel Bindungen bilden können. Bei den auf dem Markt vorherrschenden Superabsorbern auf Acrylsäure/Natriumacrylat-Basis sind geeignete Oberflächennachvernetzer Verbindungen, die Gruppen enthalten, die mit mindestens zwei Carboxylatgruppen Bindungen bilden können. Statt "Oberflächennachvernetzer" oder "Oberfächennachvernetzung" wird oft auch nur "Nachvernetzer" oder "Nachvernetzung" verwendet.

Bevorzugte Oberflächennachvernetzer sind Di- oder Triglycidylverbindungen wie beispielsweise Glycidylether, etwa Ethylenglykoldiglycidylether, Glyzerindi- oder -triglycidylether.

Bevorzugte Oberflächennachvernetzer sind auch 2-Oxazolidone, wie 2-Oxazolidon und N-(2-Hydroxiethyl)-2-oxazolidon, N-Methyl-2-Oxazolidon, N-Acyl-2-oxazolidone, wie N-Acetyl-2-oxazolidon, 2-Oxotetrahydro-1,3-oxazin, bicyclische Amidacetale, wie 5-Methyl-1-aza-4,6-dioxa-bicyclo[3.3.0]octan, 1-Aza-4,6-dioxa-bicyclo[3.3.0]octan und 5-Isopropyl-1-aza-4,6-dioxa-bicyclo [3.3.0] octan, Bis-2-oxazolidone und Poly-2-oxazolidone. Unter diesen sind besonders bevorzugt 2-Oxazolidon, N-Methyl-2-oxazolidon, N-(2-Hydroxiethyl)-2-oxazolidon und N-Hydroxipropyl-2-oxazolidon.

Weitere bevorzugte Nachvernetzer sind 1,3-Propandiol, 1,5-Pentandiol, 1,6-Hexandiol und 1,7-Heptandiol, 1,3-Butandiol, 1,8-Octandiol, 1,9-Nonandiol und 1,10-Decandiol. Unter diesen sind besonders bevorzugt diejenigen, die sich bei 23°C zu mindestens 30 Gew.-%, bevorzugt zu mindestens 40 Gew.-%, besonders bevorzugt zu mindestens 50 Gew.-%, ganz besonders bevorzugt mindestens zu 60 Gew.-%, in Wasser lösen, wie beispielsweise 1,3-Propandiol und 1,7-Heptandiol. Noch mehr bevorzugt sind solche, die bei 25°C flüssig sind.

Weitere bevorzugte Nachvernetzer sind Butan-1,2,3-triol, Butan-1,2,4-triol, Glyzerin, Trimethylolpropan, Trimethylolethan, Pentaerythrit, pro Molekül 1- bis 3-fach ethoxyliertes Glyzerin, Trimethylolethan oder Trimethylolpropan und pro Molekül 1- bis 3-fach propoxyliertes Glyzerin, Trimethylolethan oder Trimethylolpropan. Weiterhin bevorzugt sind 2-fach ethoxyliertes oder propoxyliertes Neopentylglykol. Besonders bevorzugt sind 2-fach und 3-fach ethoxyliertes Glyzerin, Neopentylglykol, 2-Methyl-1,3-propandiol und Trimethylolpropan. Unter diesen besonders bevorzugt sind diejenigen, die bei 23 °C eine Viskosität von weniger als 3000 mPas, vorzugsweise weniger als 1500 mPas, bevorzugt weniger als 1000 mPas, besonders bevorzugt weniger als 500 mPas, ganz besonders bevorzugt weniger als 300 mPas, aufweisen.

Weitere bevorzugte Nachvernetzer sind Ethylencarbonat und Propylencarbonat.

Ein weiterer bevorzugter Nachvernetzer ist 2,2'-Bis(2-oxazolin).

Diese bevorzugten Nachvernetzer minimieren Neben- und Folgereaktionen, die zu flüchtigen und damit übelriechenden Verbindungen führen. Die mit den bevorzugten Nachvernetzern hergestellten Superabsorber sind daher auch im angefeuchteten Zustand geruchsneutral.

Es kann ein einzelner Nachvernetzer verwendet werden oder beliebige Gemische verschiedener Nachvernetzer.

Der Nachvernetzer wird im Allgemeinen in einer Menge von mindestens 0,001 Gew.-%, vorzugsweise von mindestens 0,02 Gew.-%, in besonders bevorzugter Form von mindestens 0,05 Gew.-% sowie im Allgemeinen höchstens 2 Gew.-%, vorzugsweise höchstens 1 Gew.-%, in besonders bevorzugter Form höchstens 0,3 Gew.-%, beispielsweise höchstens 0,15 Gew.-% oder höchstens 0,095 Gew.-% eingesetzt, jeweils auf die Masse des damit beaufschlagten Grundpolymeren (beispielsweise der betreffenden Siebfraktion) bezogen.

Die Nachvernetzung wird üblicherweise so durchgeführt, dass eine Lösung des Nachvernetzers auf die getrockneten Grundpolymerpartikel aufgesprüht wird. Im Anschluss an das Aufsprühen werden die mit Nachvernetzer beschichteten Polymerpartikel thermisch getrocknet, wobei die Nachvernetzungsreaktion sowohl vor als auch während der Trocknung stattfinden kann. Falls Oberflächennachvernetzer mit polymerisierbaren Gruppen verwendet werden, kann die Oberflächennachvernetzung auch durch radikalisch induzierte Polymerisation solcher Gruppen mittels gängiger Radikalbildner oder auch mittels energiereicher Strahlung wie beispielsweise UV-Licht erfolgen. Dies kann parallel oder anstatt der Verwendung von Nachvernetzern erfolgen, die kovalente oder ionische Bindungen zu funktionellen Gruppen an der Oberfläche der Grundpolymerpartikel ausbilden.

Das Aufsprühen der Nachvernetzerlösung wird vorzugsweise in Mischern mit bewegten Mischwerkzeugen, wie Schneckenmischern, Scheiben-, Paddel- oder Schaufelmischern oder Mischern mit anderen Mischwerkzeugen durchgeführt. Besonders bevorzugt sind jedoch Vertikalmischer. Es ist aber auch möglich die Nachvernetzerlösung in einem Wirbelbett aufzusprühen. Geeignete Mischer sind beispielsweise als Pflugschar®-Mischer von Gebr. Lödige Maschinenbau GmbH, Elsener-Straße 7 - 9, 33102 Paderborn, Deutschland, oder als Schugi® Flexomix®-Mischer, Vrieco-Nauta®-Mischer oder Turbulizer®-Mischer von Hosokawa Micron BV, Gildenstraat 26, 7000 AB Doetinchem, Niederlande, erhältlich.

Die einsetzbaren Sprühdüsen unterliegen keiner Beschränkung. Geeignete Düsen und Zerstäubungssysteme sind beispielsweise in den folgenden Literaturstellen beschrieben: Zerstäuben von Flüssigkeiten, Expert-Verlag, Bd. 660, Reihe Kontakt & Studium, Thomas Richter (2004) sowie in Zerstäubungstechnik, Springer-Verlag, VDI-Reihe, Günter Wozniak (2002). Einsetzbar sind mono- und polydisperse Sprühsysteme. Unter den polydispersen Systemen sind Einstoff-Druckdüsen (strahl- oder lamellenbildend), Rotationszerstäuber, Zweistoffzerstäuber, Ultraschallzerstäuber und Pralldüsen geeignet. Bei den Zweistoffzerstäubern kann die Mischung der Flüssigkeits- mit der Gasphase sowohl innenliegend als auch außenliegend erfolgen. Das Sprühbild der Düsen ist unkritisch und kann jede beliebige Form annehmen, beispielsweise Rundstrahl-, Flachstrahl-, Weitwinkel-Rundstrahl- oder Kreisring-Spritzbild. Vorteilhaft ist die Verwendung eines nicht-oxidierenden Gases, falls Zweistoffzerstäuber eingesetzt werden, besonders bevorzugt sind Stickstoff, Argon oder Kohlendioxid. Derartigen Düsen kann die zu versprühende Flüssigkeit unter Druck zugeführt werden. Die Zerteilung der zu versprühenden Flüssigkeit kann dabei dadurch erfolgen, dass sie nach Erreichen einer bestimmten Mindestgeschwindigkeit in der Düsenbohrung entspannt wird. Ferner können für den erfindungsgemäßen Zweck auch Einstoffdüsen, wie beispielsweise Schlitzdüsen oder Drallkammern (Vollkegeldüsen) verwendet werden (beispielsweise von Düsen-Schlick GmbH, DE, oder von Spraying Systems Deutschland GmbH, DE). Derartige Düsen sind auch in der EP 0 534 228 A1 und der EP 1 191 051 A2 beschrieben.

Die Nachvernetzer werden typischerweise als wässrige Lösung eingesetzt. Wird ausschließlich Wasser als Lösungsmittel verwendet, so wird der Nachvernetzerlösung oder bereits dem Grundpolymer vorteilhafterweise ein Tensid oder Deagglomerisationshilfsmittel zugesetzt. Dadurch wird das Benetzungsverhalten verbessert und die Verklumpungsneigung vermindert.

Alle anionischen, kationischen, nichtionischen und amphoteren Tenside sind als Deagglomerationshilfsmittel geeignet, bevorzugt sind jedoch aus Hautverträglichkeitsgründen nicht-ionische und amphotere Tenside. Das Tensid kann auch Stickstoff enthalten. Beispielsweise werden Sorbitanmonoester, wie Sorbitanmonococoat und Sorbitanmonolaurat, oder ethoxylierte Varianten davon, wie beispielsweise Polysorbat 20®, zugesetzt. Weitere geeignete Deagglomerationshilfsmittel stellen die ethoxylierten und alkoxylierten Derivate des 2-Propylheptanols dar, die unter den Marken Lutensol XL® und Lutensol XP® vertrieben werden (BASF SE, Carl-Bosch-Straße 38, 67056 Ludwigshafen, Deutschland).

Das Deagglomerationshilfsmittel kann getrennt dosiert oder der Nachvernetzerlösung zugesetzt werden. Vorzugsweise wird das Deagglomerationshilfsmittel der Nachvernetzerlösung einfach zugesetzt.

Die Einsatzmenge des Deagglomerationshilfsmittels bezogen auf Grundpolymer beträgt beispielsweise 0 bis 0,1 Gew.-%, vorzugsweise 0 bis 0,01 Gew.-%, besonders bevorzugt 0 bis 0,002 Gew.-%. Vorzugsweise wird das Deagglomerationshilfsmittel so dosiert, dass die Oberflächenspannung eines wässrigen Extrakts des gequollenen Grundpolymers und/oder des gequollenen nachvernetzten wasserabsorbierenden Polymeren bei 23 °C mindestens 0,060 N/m, vorzugsweise mindestens 0,062 N/m, besonders bevorzugt mindestens 0,065 N/m, und vorteilhaft höchstens 0,072 N/m beträgt.

Die wässrige Nachvernetzerlösung kann neben dem mindestens einen Nachvernetzer auch noch ein Cosolvens enthalten. Über den Gehalt an nichtwässrigem Lösungsmittel bzw. Gesamtlösungsmittelmenge kann die Eindringtiefe des Nachvernetzers in die Polymerpartikel eingestellt werden. Technisch gut geeignete Cosolventien sind C1-C6-Alkohole, wie Methanol, Ethanol, n-Propanol, Isopropanol, n-Butanol, sec-Butanol, tert.-Butanol oder 2-Methyl-1-propanol, $C_2$-$C_5$-Diole, wie Ethylenglykol, 1,2-Propylenglykol oder 1,4-Butandiol, Ketone, wie Aceton, oder Carbonsäureester, wie Essigsäureethylester. Nachteilig an einigen dieser Cosolventien ist, dass sie typische Eigengerüche aufweisen.

Das Cosolvens selbst ist unter den Reaktionsbedingungen idealerweise kein Nachvernetzer. Es kann jedoch im Grenzfall und abhängig von Verweilzeit und Temperatur dazu kommen, dass das Cosolvens teilweise zur Vernetzung beiträgt. Dies ist insbesondere dann der Fall, wenn der Nachvernetzer relativ träge ist und daher auch selbst sein Cosolvens bilden kann, wie beispielsweise bei Einsatz cyclischer Carbonate, Diole oder Polyole. Solche Nachvernetzer können im Gemisch mit reaktiveren Nachvernetzern auch in der Funktion als Cosolvens eingesetzt werden, da die eigentliche Nachvernetzungsreaktion dann bei niedrigeren Temperaturen und/oder kürzeren Verweilzeiten als in Abwesenheit des reaktiveren Vernetzers durchgeführt werden kann. Da das Cosolvens in relativ großen Mengen verwendet wird und auch teilweise im Produkt verbleibt, darf es nicht toxisch sein.

Im erfindungsgemäßen Verfahren eignen sich die oben genannten Diole, Polyole, sowie die cyclischen Carbonate auch als Cosolventien. Diese Funktion erfüllen sie in Gegenwart eines reaktiveren Nachvernetzers und/oder einer Di- oder Triglycidylverbindung. Bevorzugte Cosolventien im erfindungsgemäßen Verfahren sind jedoch insbesondere die genannten Diole, insbesondere, wenn die Hydroxigruppen sterisch durch Nachbargruppen an einer Reaktion behindert werden. Solche Diole eignen sich zwar prinzipiell auch als Nachvernetzer, erfordern dazu jedoch deutlich höhere Reaktionstemperaturen oder gegebenenfalls höhere Einsatzmengen als sterisch ungehinderte Diole.

Besonders bevorzugte Kombinationen aus wenig reaktivem Nachvernetzer als Cosolvens und reaktivem Nachvernetzer sind Kombinationen von genannten mehrwertigen Alkoholen, Diolen und Polyolen mit den genannten Amidacetalen oder Carbamaten. Geeignete Kombinationen sind beispielsweise 2-Oxazolidon/1,2-Propandiol und N-(2-Hydroxiethyl)-2-oxazolidon/1,2-Propandiol sowie Ethylenglykoldiglycidylether/1,2-Propandiol. Ganz besonders bevorzugte Kombinationen sind 2-Oxazolidon/1,3-Propandiol und N-(2-Hydroxiethyl)-2-oxazolidon/1,3-Propandiol. Weiterhin bevorzugte Kombinationen sind solche mit Ethylenglykoldiglycidylether oder Glyzerindi- oder -triglycidylether mit folgenden Lösemitteln, Cosolventien oder Covernetzern: Isopropanol, 1,3-Propandiol, 1,2-Propylenglykol oder Gemischen davon. Weiterhin bevorzugte Kombinationen sind solche mit 2-Oxazolidon oder (2-Hydroxiethyl)-2-oxazolidon in folgenden Lösemitteln, Cosolventien oder Covernetzern: Isopropanol, 1,3-Propandiol, 1,2-Propylenglykol, Ethylencarbonat, Propylencarbonat oder Gemischen davon.

Häufig beträgt die Konzentration des Cosolvens in der wässrigen Nachvernetzerlösung, von 15 bis 50 Gew.-%, vorzugsweise von 15 bis 40 Gew.-%, besonders bevorzugt von 20 bis 35 Gew.-%, bezogen auf die Nachvernetzerlösung. Bei Cosolventien, die mit Wasser nur begrenzt mischbar sind, wird man vorteilhaft die wässrige Nachvernetzerlösung so einstellen, dass nur eine Phase vorliegt, gegebenenfalls durch Erniedrigung der Konzentration des Cosolvens.

In einer bevorzugten Ausführungsform wird kein Cosolvens eingesetzt. Der Nachvernetzer wird dann nur als Lösung in Wasser angewandt, gegebenenfalls unter Zusatz eines Deagglomerationshilfsmittels.

Die Konzentration des mindestens einen Nachvernetzers in der wässrigen Nachvernetzerlösung, beträgt typischerweise 1 bis 20 Gew.-%, vorzugsweise 1,5 bis 10 Gew.-%, besonders bevorzugt 2 bis 5 Gew.-%, bezogen auf die Nachvernetzerlösung.

Die Gesamtmenge der Nachvernetzerlösung bezogen auf Grundpolymer beträgt üblicherweise von 0,3 bis 15 Gew.-%, vorzugsweise von 2 bis 6 Gew.-%.

Die eigentliche Oberflächennachvernetzung durch Reaktion des Oberflächennachvernetzers mit funktionellen Gruppen an der Oberfläche der Grundpolymerpartikel wird meist durch Erwärmung des mit Oberflächennachvernetzerlösung benetzten Grundpolymers durchgeführt, üblicherweise "Trocknung" genannt (aber nicht mit der oben beschriebenen Trocknung des Polymergels aus der Polymerisation zu verwechseln, bei der typischerweise sehr viel mehr Flüssigkeit zu entfernen ist). Die Trocknung kann im Mischer selbst erfolgen, durch Beheizung des Mantels, durch Wärmeaustauschflächen oder Einblasen warmer Gase. Gleichzeitiges Versetzen des Superabsorbers mit Oberflächennachvernetzer und Trocknen kann beispielsweise in einem Wirbelschichttrockner erfolgen. Die Trocknung wird aber meist in einem nachgeschalteten Trockner, wie beispielsweise einem Hordentrockner, einem Drehrohrofen, ein Paddel- oder Scheibentrockner oder einer beheizbaren Schnecke durchgeführt. Geeignete Trockner sind beispielsweise als Solidair® oder Torusdisc®-Trockner von Bepex International LLC, 333 N.E. Taft Street, Minneapolis, MN 55413, U.S.A., oder als Paddel- oder Schaufeltrockner oder auch als Fließbetttrockner von Nara Machinery Co., Ltd., Zweigniederlassung Europa, Europaallee 46, 50226 Frechen, Deutschland erhältlich.

Es ist möglich die Polymerpartikel zur Trocknung und Durchführung der Oberflächennachvernetzung über Kontaktflächen in einem nachgeschalteten Trockner zu beheizen, oder über zugeführtes warmes Inertgas, oder über eine Mischung eines oder mehrerer Inertgase mit Wasserdampf, oder nur mit Wasserdampf allein. Bei Zufuhr der Wärme über Kontaktflächen ist es möglich die Reaktion bei leichtem oder vollständigem Unterdruck unter Inertgas durchzuführen. Bei Verwendung von Wasserdampf zum direkten Beheizen der Polymerpartikel ist es erfindungsgemäß wünschenswert den Trockner bei Normaldruck oder Überdruck zu betreiben. In diesem Fall kann es sinnvoll sein den Nachvernetzungsschritt in einen Aufheizschritt mit Wasserdampf und einen Reaktionsschritt unter Inertgas aber ohne Wasserdampf aufzuspalten. Dies kann in einem oder mehreren Apparaten realisiert werden. Erfindungsgemäß können die Polymerpartikel schon im Nachvernetzungsmischer mit Wasserdampf aufgeheizt werden. Das eingesetzte Grundpolymer kann aus vorhergehenden Prozessschritten noch eine Temperatur von 10 bis 120 °C aufweisen, die Nachvernetzerlösung kann eine Temperatur von 0 bis 70 °C aufweisen. Insbesondere kann die Nachvernetzerlösung zur Verminderung der Viskosität erwärmt werden.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten. Typischerweise wird die Trocknung so geführt, dass der Superabsorber einen Restfeuchtegehalt von im Allgemeinen mindestens 0,1 Gew.-%, vorzugsweise mindestens 0,2 Gew.-% und in besonders bevorzugter Form mindestens 0,5 Gew.-% sowie im Allgemeinen höchstens 15 Gew.-%, vorzugsweise höchstens 10 Gew.-% und in besonders bevorzugter Form höchstens 8 Gew.-% aufweist.

Die Nachvernetzung kann unter normalen atmosphärischen Bedingungen stattfinden. Normale atmosphärische Bedingungen bedeutet, dass keine technischen Vorkehrungen getroffen werden, um den Partialdruck oxidierender Gase wie den des atmosphärischen Sauerstoffs im Apparat, in dem die Nachvernetzungsreaktion überwiegend stattfindet (dem "Nachvernetzungsreaktor", typischerweise der Trockner) zu verringern. Es ist jedoch bevorzugt, die Nachvernetzungsreaktion unter verringertem Partialdruck oxidierender Gase durchzuführen. Oxidierende Gase sind Stoffe, die bei 23 °C einen Dampfdruck von mindestens 1013 mbar aufweisen und in Verbrennungsvorgängen als Oxidationsmittel wirken, beispielsweise Sauerstoff, Stickoxid und Stickstoffdioxid, insbesondere Sauerstoff. Vorzugsweise beträgt der Partialdruck oxidierender Gase dabei weniger als 140 mbar, bevorzugt weniger als 100 mbar, besonders bevorzugt weniger als 50 mbar, ganz besonders bevorzugt weniger als 10 mbar. Wird die thermische Nachvernetzung bei Umgebungsdruck, d. h. bei einem Gesamtdruck um 1013 mbar, durchgeführt, so wird der Gesamtpartialdruck der oxidierenden Gase über deren Volumenanteil festgelegt. Der Anteil der oxidierenden Gase beträgt dabei vorzugsweise weniger als 14 Vol.-%, bevorzugt weniger als 10 Vol.-%, besonders bevorzugt weniger als 5 Vol.-%, ganz besonders bevorzugt weniger als 1 Vol.-%.

Die Nachvernetzung kann unter vermindertem Druck durchgeführt werden, d. h. bei einem Gesamtdruck von weniger als 1.013 mbar. Der Gesamtdruck beträgt typischerweise weniger als 670 mbar, vorzugsweise weniger als 480 mbar, besonders bevorzugt weniger als 300 mbar, ganz besonders bevorzugt weniger als 200 mbar. Werden Trocknung und Nachvernetzung unter Luft mit einem Sauerstoffgehalt von 20,8 Vol.-% durchgeführt, so betragen die zu den obengenannten Gesamtdrücken korrespondierenden Sauerstoffpartialdrücke 139 mbar (670 mbar), 100 mbar (480 mbar), 62 mbar (300 mbar) und 42 mbar (200 mbar), wobei die jeweiligen Gesamtdrücke in den Klammern stehen. Eine andere Möglichkeit, den Partialdruck oxidierender Gase zu senken, ist die Einleitung von nicht oxidierenden Gasen, insbesondere Inertgasen in den zur Nachvernetzung verwendeten Apparat. Geeignete Inertgase sind bei der Nachvernetzungstemperatur und gegebenem Druck im Nachvernetzungstrockner gasförmig vorliegende Stoffe, die unter diesen Bedingungen nicht oxidierend auf die Bestandteile der trocknenden Polymerpartikel wirken, beispielsweise Stickstoff, Kohlendioxid, Argon, Wasserdampf, wobei Stickstoff bevorzugt ist. Die Inertgasmenge beträgt im Allgemeinen von 0,0001 bis 10 m³, bevorzugt 0,001 bis 5 m³, besonders bevorzugt 0,005 bis 1 m³, und ganz besonders bevorzugt 0,005 bis 0,1 m³, bezogen auf 1 kg Superabsorber.

Im erfindungsgemäßen Verfahren kann das Inertgas, wenn es nicht Wasserdampf enthält, über Düsen in den

**EP 3 706 900 B1**

Nachvernetzungstrockner eingeblasen werden, besonders bevorzugt wird das Inertgas aber bereits im oder kurz vor dem Mischer, indem der Superabsorber mit Oberflächennachvernetzer versetzt wird, über Düsen dem Polymerpartikelstrom zugegeben.

Selbstverständlich können aus dem Trockner abgeführte Dämpfe von Cosolventien außerhalb des Trockners wieder kondensiert und gegebenenfalls rezykliert werden.

Auf die Oberflächen des erfindungsgemäßen Superabsorbers werden vor, während oder nach der Nachvernetzung zusätzlich zu den genannten, kovalente Bindungen mit Carboxigruppen des Superabsorbers ausbildenden organischen Nachvernetzern Aluminiumionen aufgebracht oder, falls keine Oberflächennachvernetzung mit einem der genannten organischen Nachvernetzer durchgeführt wird, anstatt dieser. Wie oben bereits gesagt, ist dieses Aufbringen von Aluminiumionen im Prinzip eine (gegebenenfalls zusätzliche) Oberflächennachvernetzung durch ionische, nicht kovalente Bindungen und wird im Rahmen dieser Erfindung zur Unterscheidung von Oberflächennachvernetzung mittels kovalenter Bindungen als "Komplexierung" mit den betreffenden Metallionen bezeichnet.

Dieses Aufbringen von Aluminiumionen erfolgt durch Zugabe einer wässrigen, Aluminiumionen enthaltenden Lösung, die sich dadurch auszeichnet, dass sie Aluminiumionen in einem Anteil im Bereich von 0,5 - 15 Gewichts-% (ggf. umgerechnet auf $Al^{3+}$) bezogen auf die Gesamtmasse der Lösung umfasst und weiterhin Anionen der Milchsäure (Laktat-Ionen) und der Phosphorsäure (Phosphat-Ionen) umfasst, wobei der molare Anteil der Laktat-Ionen im Bereich des 0,01 - 2,99-fachen bezogen auf den molaren Anteil an $Al^{3+}$ ist und der molare Anteil der Phosphatlonen im Bereich des 0,01 - 2,99-fachen bezogen auf den molaren Anteil an $Al^{3+}$ ist.

Die Zugabe erfolgt vorzugsweise so, dass die Oberflächen der Superabsorberpartikel des Grundpolymers oder des bereits oberflächennachvernetzten Polymers gleichmäßig mit der Lösung benetzt werden. Grundsätzlich erfolgt dies in gleicher Weise wie für den organischen Oberflächennachvernetzer beschrieben, einschließlich des Trocknungsschritts. Das Aufsprühen der Aluminiumlösung auf die Superabsorberpartikel kann sowohl vor als auch nach der Oberflächennachvernetzung erfolgen. In einem besonders bevorzugten Verfahren erfolgt das Aufsprühen der Aluminiumlösung im gleichen Schritt mit dem Aufsprühen der Vernetzer-Lösung, wobei beide Lösungen getrennt nacheinander oder gleichzeitig über zwei Düsen aufgesprüht werden, oder Vernetzer- und Metallsalz-Lösung vereint über eine Düse aufgesprüht werden können.

Sofern im Anschluss an die Oberflächennachvernetzung und/oder Behandlung mit Komplexbildner ein Trocknungsschritt durchgeführt wird, ist es vorteilhaft, aber nicht unbedingt notwendig, das Produkt nach der Trocknung zu kühlen. Die Kühlung kann kontinuierlich oder diskontinuierlich erfolgen, bequemerweise wird das Produkt dazu kontinuierlich in einen dem Trockner nachgeschalteten Kühler gefördert. Dazu kann jeder zur Abfuhr von Wärme aus pulverförmigen Feststoffen bekannte Apparat verwendet werden, insbesondere jede oben als Trocknungsapparat erwähnte Vorrichtung, sofern sie nicht mit einem Heizmedium, sondern mit einem Kühlmedium wie etwa mit Kühlwasser beaufschlagt wird, so dass über die Wände und je nach Konstruktion auch über die Rührorgane oder sonstige Wärmeaustauschflächen keine Wärme in den Superabsorber eingetragen, sondern daraus abgeführt wird. Bevorzugt ist die Verwendung von Kühlern, in denen das Produkt bewegt wird, also gekühlten Mischern. beispielsweise Schaufelkühlern, Scheibenkühlern oder Paddelkühlern. Der Superabsorber kann auch in der Wirbelschicht durch Einblasen eines gekühlten Gases wie kalter Luft gekühlt werden. Die Bedingungen der Kühlung werden so eingestellt, dass ein Superabsorber mit der für die Weiterverarbeitung gewünschten Temperatur erhalten wird. Typischerweise wird eine mittlere Verweilzeit im Kühler von im Allgemeinen mindestens 1 Minute, vorzugsweise mindestens 3 Minuten und in besonders bevorzugter Form mindestens 5 Minuten sowie im Allgemeinen höchstens 6 Stunden, vorzugsweise höchstens 2 Stunden und in besonders bevorzugter Weise höchstens 1 Stunde eingestellt und die Kühlleistung so bemessen, dass das erhaltene Produkt eine Temperatur von im Allgemeinen mindestens 0 °C, vorzugsweise mindestens 10 °C und in besonders bevorzugter Form mindestens 20 °C sowie im Allgemeinen höchstens 100 °C, vorzugsweise höchstens 80 °C und in besonders bevorzugter Form höchstens 60 °C aufweist.

Der oberflächennachvernetzte Superabsorber wird wahlweise in üblicher Weise gemahlen und/oder gesiebt. Mahlung ist hier typischerweise nicht erforderlich, meist ist aber zur Einstellung der gewünschten Partikelgrößenverteilung des Produkts das Absieben von gebildeten Agglomeraten oder Feinkorn angebracht. Agglomerate und Feinkorn werden entweder verworfen oder vorzugsweise in bekannter Weise und an geeigneter Stelle in das Verfahren zurückgeführt; Agglomerate nach Zerkleinerung. Die für oberflächennachvernetzte Superabsorber gewünschten Partikelgrößen sind die gleichen wie bei Grundpolymeren.

Wahlweise werden die mit dem erfindungsgemäßen Verfahren hergestellten erfindungsgemäßen Superabsorber mit weiteren Zusätzen versehen, als nicht einschränkende Beispiele solchen, die gegen Verfärbung stabilisieren, die Verbackungsneigung verringern oder die Permeabilität weiter erhöhen. Hierzu können alle bekannten Zusätze auf die für sie jeweils bekannte Weise im erfindungsgemäßen Verfahren eingesetzt werden. Beispiele von bekannten, gegen Verfärbung stabilisierenden Zusätzen sind die oben genannten Sulfonsäure- oder Phosphonsäurederivate, die statt oder zusätzlich zur Zugabe während der Herstellung des erfindungsgemäßen Superabsorbers auch nach seiner Herstellung aufgebracht werden können. Beispiele für bekannte Zusätze, welche die Verbackungsneigung des Superabsorbers verringern oder die Permeabilität weiter erhöhen, sind wasserunlösliche anorganische Pulver.

Vorzugweise wird dem erfindungsgemäßen Superabsorber ein wasserunlösliches anorganisches Pulver zugesetzt. Bevorzugterweise werden gefälltes oder durch Pyrolyse hergestelltes Siliciumdioxid sowie durch Pyrolyse hergestelltes Aluminiumoxid verwendet. Pyrogenes Siliciumdioxid ist beispielsweise unter der Marke AEROSIL® und pyrogenes Aluminiumoxid beispielsweise unter der Marke AEROXIDE® Alu von Evonik Industries AG, Inorganic Materials, Rodenbacher Chaussee 4, 63457 Hanau-Wolfgang, Deutschland, erhältlich. Durch Fällung erzeugtes Siliciumdioxid ist beispielsweise unter der Marke SIPERNAT® von Evonik Industries AG, Inorganic Materials, Rodenbacher Chaussee 4, 63457 Hanau-Wolfgang, Deutschland, erhältlich. Die wasserunlöslichen anorganischen Pulver können durch geeignete Oberflächenbehandlung auch hydrophobiert werden und werden von Herstellern oft sowohl in hydrophobierter als auch in hydrophiler Form angeboten. Im Rahmen dieser Erfindung ist die Verwendung hydrophiler wasserunlöslicher anorganischer Pulver bevorzugt.

Im Allgemeinen wird das wasserunlösliche anorganische Pulver dem Superabsorber in einer Menge von mindestens 0,005 Gew.-%, vorzugsweise von mindestens 0,03 Gew.-% und in besonders bevorzugter Form von mindestens 0,05 Gew.-% sowie im Allgemeinen von höchstens 6,0 Gew.-%, vorzugsweise höchstens 1,0 Gew.-% und in besonders bevorzugter Form höchstens 0,5 Gew.-% zugegeben, jeweils bezogen auf das Gesamtgewicht des wasserfreien Superabsorbers mit anorganischem Pulver.

Die Vermischung von Superabsorbern mit den optionalen Zusätzen kann durch jedes bekannte Mischverfahren erfolgen. Sie werden, sofern in fester Form, in Substanz oder als Suspension in einem Lösungs- oder Suspensionsmittel eingemischt, sofern in gelöster oder flüssiger Form, wahlweise auch in Lösung oder flüssiger Form. Vorzugsweise werden die Zusätze aufgrund der leichteren homogenen Verteilung dem Superabsorber als Pulver oder Suspension eingemischt. Dabei wird nicht notwendigerweise eine einfach durch mechanische Maßnahmen trennbare physikalische Mischung erzeugt. Die Zusatzstoffe können durchaus eine festere Verbindung mit dem Superabsorber eingehen, beispielsweise als vergleichsweise fest anhaftende Oberflächenschicht oder als fest an der Oberfläche der Superabsorberpartikel anhaftende Partikel. Die Einmischung der Zusatzstoffe in den bekannten Superabsorber kann durchaus auch als "Beschichtung" verstanden und bezeichnet werden.

Falls zur Beschichtung eine Lösung oder Suspension verwendet wird, wird als Lösungs- oder Suspensionsmittel ein Lösungs- oder Suspensionsmittel verwendet, das sowohl mit dem Superabsorber wie auch mit dem Zusatzstoff chemisch verträglich ist, also keine unerwünschten chemischen Reaktionen damit eingeht. Typischerweise wird Wasser oder ein organisches Lösungsmittel verwendet, beispielsweise ein Alkohol oder Polyol, oder Mischungen davon. Beispiele geeigneter Lösungs- oder Suspensionsmittel sind Wasser, Isopropanol/Wasser, 1,3-Propandiol/Wasser und Propylenglykol/Wasser, wobei das Mischungsmassenverhältnis vorzugsweise von 20:80 bis 40:60 beträgt. Wird ein Suspensionsmittel für die erfindungsgemäß zu verwendenden Stabilisatoren oder den anorganischen partikulären Feststoff verwendet, so ist Wasser bevorzugt. Der Lösung oder Suspension kann ein Tensid zugesetzt werden.

Optionale Zusatzstoffe werden, sofern sie nicht der Monomermischung oder dem polymerisierenden Gel zugegeben werden, im Allgemeinen auf genau die gleiche Weise mit dem Superabsorber vermischt wie die zur Oberflächennachvernetzung auf den Superabsorber aufgebrachte, einen Oberflächennachvernetzer enthaltende Lösung oder Suspension. Der Zusatzstoff kann als Bestandteil der zur Oberflächennachvernetzung aufgebrachten Lösung oder einer ihrer Komponenten auf einen (noch) nicht nachvernetzten Superabsorber (ein "Grundpolymer" oder "Basispolymer") aufgebracht, also der Lösung des Oberflächennachvernetzers oder einer ihrer Komponenten zugesetzt werden. Der mit Oberflächennachvernetzungsmittel und Zusatzstoffen beschichtete Superabsorber durchläuft dann die weiteren zur Oberflächennachvernetzung erforderlichen Verfahrensschritte, beispielsweise eine thermisch induzierte Reaktion des Oberflächennachvernetzungsmittels mit dem Superabsorber. Dieses Verfahren ist vergleichsweise einfach und wirtschaftlich.

Falls der Superabsorber nach der Oberflächennachvernetzung oder der Komplexierung einem Kühlschritt unterworfen wird, können die wahlweisen Zusätze bequemerweise im Kühler untergemischt werden. Falls Zusatzstoffe als Lösung oder Suspension aufgebracht werden, kann die Aufbringung auch auf den bereits oberflächennachvernetzten Superabsorber in den gleichen Mischapparaten wie für die Aufbringung des Oberflächennachvernetzungsmittels auf das Grundpolymer beschrieben erfolgen. Meist, aber nicht notwendigerweise wird anschließend ebenso wie bei der Oberflächennachvernetzung erwärmt, um den Superabsorber wieder zu trocknen. Die bei dieser Trocknung eingestellte Temperatur liegt dann aber im Allgemeinen bei höchstens 110 °C, vorzugsweise höchstens 100 °C und in besonders bevorzugter Weise höchstens 90 °C, um unerwünschte Reaktionen des Zusatzstoffs zu vermeiden. Die Temperatur wird so eingestellt, dass in Anbetracht der Verweilzeit im Trocknungsaggregat der gewünschte Wassergehalt des Superabsorbers erreicht wird. Es ist durchaus auch möglich und bequem, Zusatzstoffe einzeln oder gemeinsam mit anderen üblichen Hilfsmitteln, beispielsweise Staubbindemitteln, Mitteln gegen Verbackung oder Wasser zur Rückbefeuchtung des Superabsorbers zuzugeben. Die Temperatur der Polymerpartikel beträgt in diesem Fall zwischen 0 °C und 190 °C, bevorzugt weniger als 160 °C, mehr bevorzugt weniger als 130 °C, noch mehr bevorzugt weniger als 100 °C, und am meisten bevorzugt weniger als 70 °C. Die Polymerpartikel werden gegebenenfalls nach Beschichtung zügig auf Temperaturen unterhalb einer etwaigen Zersetzungstemperatur des Zusatzstoffs abgekühlt.

Wahlweise können auf die Oberfläche der Superabsorberpartikel, ob nicht nachvernetzt oder nachvernetzt, im Herstellverfahren in jedem Prozessschritt bei Bedarf alle bekannten Beschichtungen, wie filmbildende Polymere, thermoplastische Polymere, Dendrimere, polykationische Polymere (wie beispielsweise Polyvinylamin, Polyethylenimin oder Polyallylamin), oder alle dem Fachmann bekannten wasserlöslichen mono- oder polyvalenten Metallsalze, wie beispielsweise Aluminiumsulfat, Natrium-, Kalium-, Zirkonium- oder Eisensalze zusätzlich aufgebracht werden. Beispiele für nützliche Alkalimetallsalze sind Natrium- und Kaliumsulfat, Natrium- und Kaliumlactate, -citrate, -sorbate. Dadurch können zusätzliche Effekte, beispielsweise eine verringerte Verbackungsneigung des Endprodukts oder des Zwischenprodukts im jeweiligen Prozessschritt des Herstellverfahrens, verbesserte Verarbeitungseigenschaften oder eine weiter gesteigerte Permeabilität (SFC) erreicht werden. Wenn Zusatzstoffe in Form von Dispersionen eingesetzt und aufgesprüht werden, dann werden sie bevorzugt als wässrige Dispersionen eingesetzt, und es wird bevorzugt noch zusätzlich ein Entstaubungsmittel zur Fixierung des Additivs auf der Oberfläche des Superabsorbers aufgebracht. Das Entstaubungsmittel wird dann entweder direkt der Dispersion des anorganischen pulvrigen Additivs hinzugefügt, optional kann es auch als separate Lösung vor, während, oder nach dem Auftrag des anorganischen pulvrigen Additivs durch Aufsprühen hinzugefügt werden. Am meisten bevorzugt ist das gleichzeitige Aufsprühen von Nachvernetzungsmittel, Entstaubungsmittel und pulvrigem anorganischen Additiv in der Nachvernetzung. In einer weiteren bevorzugten Verfahrensvariante wird das Entstaubungsmittel aber separat im Kühler zugegeben, beispielsweise durch Aufsprühen von oben, unten oder von der Seite. Besonders geeignete Entstaubungsmittel, die auch zur Fixierung pulvriger anorganischer Additive an der Oberfläche der wasserabsorbierenden Polymerpartikel dienen können, sind Polyethylenglykole mit einem Molekulargewicht von 400 bis 20000 g/mol, Polyglyzerin, 3- bis 100-fach ethoxylierte Polyole, wie Trimethylolpropan, Glyzerin, Sorbitol und Neopentylglykol. Besonders geeignet sind 7- bis 20-fach ethoxyliertes Glyzerin oder Trimethylolpropan, wie beispielsweise Polyol TP 70® (Perstorp, SE). Letztere haben insbesondere den Vorteil, dass sie die Oberflächenspannung eines wässrigen Extrakts der wasserabsorbierenden Polymerpartikel nur unwesentlich herabsetzen.

Es ist ebenso möglich, den erfindungsgemäßen Superabsorber durch Wasserzusatz auf einen gewünschten Wassergehalt einzustellen. Es kann auch vorteilhaft sein, den Superabsorber durch Wasserzugabe anzuquellen und danach wieder durch Trocknung auf den gewünschten Wassergehalt einzustellen.

Alle Beschichtungen, Feststoffe, Zusätze und Hilfsstoffe können jeweils in separaten Verfahrensschritten zugegeben werden, meist ist jedoch die bequemste Methode, sie - falls sie nicht während der Versetzung des Grundpolymers mit Oberflächennachvernetzungsmittel zugegeben werden - dem Superabsorber im Kühler zuzugeben, etwa durch Aufsprühen einer Lösung oder Zugabe in feinteiliger fester oder in flüssiger Form.

Die erfindungsgemäßen Superabsorber weisen im Allgemeinen eine Zentrifugenretentionskapazität (CRC, Messmethode s. unten) von mindestens 5 g/g, vorzugsweise von mindestens 10 g/g und in besonders bevorzugter Form von mindestens 20 g/g auf. Üblicherweise liegt sie für oberflächennachvernetzte Superabsorber nicht über 40 g/g, für Grundpolymere liegt sie allerdings oft höher.

Die erfindungsgemäßen Superabsorber weisen, sofern sie oberflächennachvernetzt sind, typischerweise eine Absorption unter Druck (AUL0.7psi, Messmethode s. unten) von mindestens 10 g/g, vorzugsweise mindestens 14 g/g, bevorzugt mindestens 18 g/g und ganz besonders bevorzugt mindestens 22 g/g und üblicherweise nicht über 30 g/g auf.

Ein weiterer Gegenstand der vorliegenden Erfindung sind Hygieneartikel, enthaltend erfindungsgemäße Superabsorber, vorzugsweise ultradünne Windeln, enthaltend eine absorbierende Schicht bestehend aus 50 bis 100 Gew.-%, vorzugsweise 60 bis 100 Gew.-%, bevorzugt 70 bis 100 Gew.-%, besonders bevorzugt 80 bis 100 Gew.-%, ganz besonders bevorzugt 90 bis 100 Gew.-%, erfindungsgemäßer Superabsorber, wobei die Umhüllung der absorbierenden Schicht selbstverständlich nicht berücksichtigt ist.

Ganz besonders vorteilhaft sind die erfindungsgemäßen Superabsorber auch zur Herstellung von Laminaten und Kompositstrukturen, wie sie beispielsweise in der US 2003/0181115 sowie der US 2004/0019342 beschrieben sind, geeignet. Zusätzlich zu den in beiden Schriften zur Herstellung solcher neuer absorbierenden Strukturen beschriebenen Schmelzklebern und insbesondere den in der US 2003/0181115 beschriebenen Fasern aus Schmelzklebern, an die die Superabsorberpartikel gebunden ist, eignen sich die erfindungsgemäßen Superabsorber auch zur Herstellung von vollkommen analogen Strukturen unter Verwendung von UV-vernetzbaren Schmelzklebern, welche beispielsweise als AC-Resin® (BASF SE, Ludwigshafen, Deutschland) vertrieben werden. Diese UV-vernetzbaren Schmelzkleber haben den Vorteil bereits bei 120 bis 140 °C verarbeitbar zu sein, daher sind sie mit vielen thermoplastischen Substraten besser kompatibel. Ein weiterer wesentlicher Vorteil besteht darin, dass UV-vernetzbare Schmelzkleber toxikologisch sehr unbedenklich sind und auch keine Ausdünstungen in den Hygieneartikeln verursachen. Ein ganz wesentlicher Vorteil, im Zusammenhang mit den erfindungsgemäßen Superabsorbern, ist die Eigenschaft der UV-vernetzbaren Schmelzkleber während der Verarbeitung und Vernetzung nicht zur Vergilbung zu neigen. Dies ist insbesondere von Vorteil, wenn ultradünne oder teilweise transparente Hygieneartikel hergestellt werden sollen. Die Kombination der erfindungsgemäßen Superabsorber mit UV-vernetzbaren Schmelzklebern ist daher besonders vorteilhaft. Geeignete UV-vernetzbare Schmelzkleber sind beispielsweise beschrieben in EP 0 377 199 A2, EP 0 445 641 A1, US 5,026,806, EP 0 655 465 A1 und EP 0 377 191 A2.

Der erfindungsgemäße Superabsorber kann außerdem in anderen Gebieten der Technik eingesetzt werden, bei denen Flüssigkeiten, insbesondere Wasser oder wässrige Lösungen absorbiert werden. Diese Gebiete sind beispielsweise Lagerung, Verpackung, Transport (als Bestandteile von Verpackungsmaterial für wasser- oder feuchtigkeitsempfindliche Artikel, etwa zum Blumentransport, auch als Schutz gegen mechanische Einwirkungen); Tierhygiene (in Katzenstreu); Lebensmittelverpackung (Transport von Fisch, Frischfleisch; Absorption von Wasser, Blut in Frischfisch- oder -fleischverpackungen); Medizin (Wundpflaster, wasserabsorbierendes Material für Brandverbände oder für andere nässende Wunden), Kosmetik (Trägermaterial für Pharmachemikalien und Medikamente, Rheumapflaster, Ultraschallgel, Kühlgel, Kosmetikverdicker, Sonnenschutz); Verdicker für Öl/Wasser bzw. Wasser/Öl-Emulsionen; Textilien (Feuchtigkeitsregulation in Textilien, Schuheinlagen, zur Verdampfungskühlung, etwa in Schutzkleidung, Handschuhen, Stirnbändern); chemisch-technische Anwendungen (als Katalysator für org. Reaktionen, zur Immobilisierung großer funktioneller Moleküle wie Enzymen, als Adhäsionsmittel bei Agglomerationen, Wärmespeicher, Filtrationshilfsmittel, hydrophile Komponente in Polymerlaminaten, Dispergiermittel, Verflüssiger); als Hilfsmittel beim Pulverspritzguss, im Bau- und Konstruktionswesen (Installation, in lehmbasierenden Putzen, als vibrationshemmendes Medium, Hilfsmittel bei Tunnelgrabungen in wasserreichem Untergrund, Kabelummantelung); Wasserbehandlung, Abfallbehandlung, Wasserabtrennung (Enteisungsmittel, wiederverwendbare Sandsäcke); Reinigung; Agrarindustrie (Bewässerung, Rückhaltung von Schmelzwasser und Tauniederschläge, Kompostierungszusatz, Schutz der Wälder vor Pilz-/Insektenbefall, verzögerte Freisetzung von Wirkstoffen an Pflanzen); zur Brandbekämpfung oder zum Brandschutz; Coextrusionsmittel in thermoplastischen Polymeren (z. B. zur Hydrophilierung von Mehrschichtfolien); Herstellung von Folien und thermoplastischen Formkörpern, die Wasser absorbieren können (z.B. Regen- und Tauwasser speichernde Folien für die Landwirtschaft; Superabsorber enthaltende Folien zum Frischhalten von Obst und Gemüse, die in feuchte Folien verpackt werden; Superabsorber-Polystyrol Coextrudate, beispielsweise für Lebensmittelverpackungen wie Fleisch, Fisch, Geflügel, Obst und Gemüse); oder als Trägersubstanz in Wirkstoffformulierungen (Pharma, Pflanzenschutz).

Die erfindungsgemäßen Artikel zur Absorption von Flüssigkeit unterscheiden sich von bekannten dadurch, dass sie den erfindungsgemäßen Superabsorber enthalten.

Es wurde außerdem ein Verfahren zur Herstellung von Artikeln zur Absorption von Flüssigkeit, insbesondere Hygieneartikeln gefunden, das dadurch gekennzeichnet ist, dass man bei der Herstellung des betreffenden Artikels mindestens einen erfindungsgemäßen Superabsorber einsetzt. Im Übrigen sind Verfahren zur Herstellung solcher Artikel unter Einsatz von Superabsorber bekannt.

Testmethoden

Der Superabsorber wird mit den nachfolgend beschriebenen Testmethoden geprüft.

Die nachfolgend beschriebenen, mit "NWSP" bezeichneten Standard-Testmethoden werden beschrieben in: "Nonwovens Standards Procedures", Ausgabe 2015, gemeinsam herausgegeben von EDANA (European Disposables and Nonwovens Association, Avenue Herrmann Debroux 46, 1160 Brüssel, Belgien, www.edana.org) und INDA (Association of the Nonwoven Fabrics Industry, 1100 Crescent Green, Suite 115, Cary, North Carolina 27518, U.S.A., www.inda.org). Diese Veröffentlichung ist sowohl von EDANA als auch von INDA erhältlich.

Alle nachfolgend beschriebenen Messungen sollten, wenn nicht anders angegeben, bei einer Umgebungstemperatur von 23 $\pm$ 2 °C und einer relativen Luftfeuchte von 50 $\pm$ 10 % durchgeführt werden. Die Superabsorberpartikel werden vor der Messung gut durchmischt, wenn nicht anders angegeben.

Zentrifugenretentionskapazität (CRC, Centrifuge Retention Capacity)

Die Zentrifugenretentionskapazität des Superabsorbers wird gemäß der Standard-Testmethode Nr. NWSP 241.0 R2 (15) "Gravimetric Determination of the Fluid Retention Capacity in Saline Solution after Centrifugation" bestimmt.

Absorption unter Druck (AUL0.7psi, "Absorbency Under Load of 0.7 psi)

Die Absorption unter einem Druck von 4826 Pa (0.7 psi) des Superabsorbers wird gemäß der Standard-Testmethode Nr. NWSP 242.0 R2 (15) "Gravimetric Determination of Absorption Against Pressure" bestimmt.

Feuchtegehalt des Superabsorbers (Restfeuchte, Wassergehalt)

Der Wassergehalt der Superabsorberpartikel wird gemäß der Standard-Testmethode Nr. NWSP 230.0 R2 (15) "Estimation of the Moisture Content as Weight Loss Upon Heating" bestimmt.

Permeabilität (SFC, "Saline Flow Conductivity")

Die Permeabilität einer vom Superabsorber durch Flüssigkeitsabsorption gebildeten gequollenen Gelschicht wird unter Druckbelastung von 0.3 psi (2068 Pa), wie in EP 0 640 330 A1 beschrieben, als Gel-Layer-Permeability einer gequollenen Gelschicht aus Superabsorberpartikeln bestimmt, wobei die in zuvor genannter Patentanmeldung auf Seite 19 und in Figur 8 beschriebene Apparatur dahingehend modifiziert ist, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP 0 640 330 A1. Der Durchfluss wird automatisch erfasst.

Die Permeabilität (SFC) wird wie folgt berechnet:

$$SFC\ [cm^3s/g] = (Fg(t=0)xL0)/(dxAxWP),$$

wobei Fg(t=0) der Durchfluss an NaCl-Lösung in g/s ist, der anhand einer linearen Regressionsanalyse der Daten Fg(t) der Durchflussbestimmungen durch Extrapolation gegen t=0 erhalten wird, L0 die Dicke der Gelschicht in cm, d die Dichte der NaCl-Lösung in $g/cm^3$, A die Fläche der Gelschicht in $cm^2$ und WP der hydrostatische Druck über der Gelschicht in $dyn/cm^2$ darstellt.

Permeabilität (GBP, "Gel Bed Permeability")

Die Gelbettpermeabilität wird nach der Vorschrift in der veröffentlichten Patentanmeldung Nr. US 2005/0 256 757 A1, Absätze [0061] bis [0075] gemessen.

CIE-Farbzahl (L, a, b)

Die Farbmessung wird entsprechend dem CIELAB-Verfahren (Hunterlab, Band 8, Jahrgang 1996, Heft 7, Seiten 1 bis 4) mit einem Colorimeter, Modell "LabScan XE S/N LX17309" (HunterLab, Reston, U.S.A.) durchgeführt. Dabei werden die Farben über die Koordinaten L, a und b eines dreidimensionalen Systems beschrieben. Dabei gibt L die Helligkeit an, wobei L = 0 schwarz und L = 100 weiß bedeutet. Die Werte für a und b geben die Position der Farbe auf den Farbachsen rot/grün bzw. gelb/blau an, wobei +a für rot, -a für grün, +b für gelb und -b für blau steht.

Die Farbmessung entspricht dem Dreibereichsverfahren nach DIN 5033-6.

Alterungstest

Messung 1 (anfängliche Farbe): Eine Plastikschale mit 9 cm Innendurchmesser wird mit Superabsorberpartikeln überfüllt und diese dann mit einem Messer über den Rand glatt gestrichen und die CIE-Farbzahlen bestimmt.

Messung 2 (nach Alterung): Eine Plastikschale mit 9 cm Innendurchmesser wird mit Superabsorberpartikeln überfüllt und diese dann mit einem Messer über den Rand glatt gestrichen. Die Schale wird dann offen in einen auf 70 °C temperierten Klimaschrank bei konstanter relativer Luftfeuchte von 80% gestellt. Die Schale wird nach Ablauf von 7 Tagen herausgenommen. Nach Abkühlen auf Raumtemperatur werden die CIE-Farbzahlen bestimmt. Anschließend wird die Schale wieder in den Klimaschrank gestellt und weitere 7 Tage temperiert. Anschließend werden wiederum nach Abkühlen auf Raumtemperatur die CIE-Farbzahlen bestimmt.

Beispiele

Brüggolit® FF6 ist ein Gemisch aus dem Dinatriumsalz der 2-Hydroxi-2-sulfinatoessigsäure, dem Dinatriumsalz der 2-Hydroxi-2-sulfonatoessigsäure und Natriumbisulfit und von L. Brüggemann KG, Salzstraße 131, 74076 Heilbronn, Deutschland erhältlich.

Laromer® LR 9015X ist das Triacrylat von fünfzehnfach ethoxyliertem Trimethylolpro-pan und erhältlich von BASF SE, Ludwigshafen, Deutschland. DAROCUR® 1173 ist 2-Hydroxi-2-methyl-1-phenylpropan-1-on und erhältlich von BASF Schweiz AG, Basel, Schweiz. IRGACURE® 651 ist 2,2-Dimethoxy-1,2-diphenylethan-1-on und ebenfalls erhältlich von BASF Schweiz AG, Basel, Schweiz.

Lohtragon® ALA 200 ist 20 Gew.-%ige wässrige Lösung von Aluminiumdihydroximonoacetat und erhältlich von Dr. Paul Lohmann GmbH KG, 31857 Emmerthal, Deutschland. Lohtragon® ACE ist eine dort erhältliche wässrige Lösung von Aluminiumdiyhdroximonoacetat mit einem Aluminiumgehalt von 4,2 Gew.-%. Die hier mit ihren Lot-Nummern bezeichneten Aluminiumsalzlösungen können ebenfalls von dort bezogen werden.

Der in den Beispielen bei Oberflächennachvernetzung und Komplexierung verwendete Mischer war ein Pflugschar®-Mischer Typ M5 mit Heizmantel von Gebr. Lödige Maschinenbau GmbH; Elsener Straße 7-9, 33102 Paderborn, Deutschland. Zur Messung der Temperatur des Produkts im Mischer wurde ein Thermoelement in die am Mischer dafür vorgesehene Öffnung so weit eingeführt, dass seine Spitze zwar von der beheizten Innenwand des Mischers beabstandet war und sich im Produkt befand, aber nicht von den Mischwerkzeugen erfasst werden konnte.

In den Beispielen wurden die Aluminiumsalzlösungen der folgenden Tabelle 1 verwendet. Hergestellt wurden diese durch Vorlegen von Wasser in einem Reaktionsgefäß, Zugabe von Aluminiumoxidhydrat unter Rühren, Zugabe von Milchsäure, Phosphorsäure sowie Säuren der anderen jeweils genannten Anionen und Rühren der erhaltenen Mischung. Die genannten Salzlösungen sind auch von Dr. Paul Lohmann GmbH KG, Hauptstraße 2, 31860 Emmerthal, Deutschland, erhältlich.

Tabelle 1 (alle Mengenangaben in Gew.-% bezogen auf die Lösung)

| Lot Nr. | Al | Laktat | $PO_4^{3-}$ | Andere | $OH^-$ | $H_2O$ |
|---------|-----|--------|-------------|--------------|--------|--------|
| 1085250 | 3,3 | 5,5 | 5,9 | Maleat, 8,1 | 3,0 | 74,2 |
| 1085251 | 2,7 | 8,9 | 4,8 | - | 2,3 | 80,1 |
| 1085252 | 3,7 | 10,2 | 6,5 | Citrat, 1,4 | 3,1 | 75,1 |
| 1086562 | 3,4 | 9,8 | 1,8 | Sulfat, 5,3 | 7,3 | 72,4 |
| 1086563 | 3,3 | 4,9 | 7,0 | Sulfat, 5,3 | 5,9 | 73,6 |
| 1099550 | 3,0 | 13,2 | 9,9 | - | 1,6 | 72,3 |
| 1099551 | 4,1 | - | 16,7 | Sulfat, 12,7 | 2,5 | 64,0 |
| 1099552 | 3,0 | - | 11,4 | Sulfat, 17,3 | 1,6 | 66,7 |

Beispiel 1

1 kg Superabsorber-Grundpolymer (HySorb® T 9600 Grundpolymer, nicht oberflächennachvernetztes vernetztes Polymer aus Acrylsäure und Natriumacrylat mit Neutralisationsgrad 72 mol-%, erhältlich von BASF SE, Ludwigshafen, Deutschland), wurde in einem Mischer vorgelegt. Bei 40 °C und einer Wellendrehzahl von 200 Umdrehungen pro Minute wurde mittels einer Zweistoff-Sprühdüse eine Lösung aus 10 g 1,2-Propandiol, 1 g einer Mischung aus gleichen Gewichtsteilen 2-Hydroxyethyloxazolidinon (HEONON) und 1,3-Propandiol, sowie 40 g der Aluminiumsalzlösung Lot # 1085250 aufgesprüht. Anschließend wurde die Wellendrehzahl auf 70 Umdrehungen pro Minute reduziert, die Produkttemperatur binnen 5 bis 10 Minuten auf 180 °C erhöht und anschließend für 80 Minuten gehalten.

Während dieser Zeit wurde jeweils nach 20, 30, 40, 50, 60 und 70 Minuten der Mischer kurz angehalten und eine Probe von ca. 10 g Produkt entnommen. Alle Proben sowie die Restmenge des Produkts nach 80 Minuten wurden auf Raumtemperatur abkühlen gelassen. Das fertige Produkt wurde durch Siebung auf den Partikelgrößenbereich von 150 μm bis 710 μm gewonnen. Die in Tabelle 2 angegebenen Messwerte wurden an den Proben und am so abgesiebten fertigen Produkt gemessen.

Beispiel 2

Beispiel 1 wurde wiederholt, wobei jedoch die Lösung Lot # 1085250 mit Lösung Lot # 1085251 ersetzt wurde. Die erhaltenen Messwerte sind in Tabelle 2 angegeben.

Beispiel 3

Beispiel 1 wurde wiederholt, wobei jedoch die Lösung Lot # 1085250 mit Lösung Lot # 1085252 ersetzt wurde. Die erhaltenen Messwerte sind in Tabelle 2 angegeben.

Beispiel 4

Beispiel 1 wurde wiederholt, wobei jedoch die Lösung Lot # 1085250 mit Lösung Lot # 1085262 ersetzt wurde. Die erhaltenen Messwerte sind in Tabelle 2 angegeben.

Beispiel 5

Beispiel 1 wurde wiederholt, wobei jedoch die Lösung Lot # 1085250 mit Lösung Lot # 1085263 ersetzt wurde. Die erhaltenen Messwerte sind in Tabelle 2 angegeben.

Auswertung

Die Beispiele 1 bis 5 zeigen, wie über die Reaktionsdauer der Komplexierung die Gelsteifigkeit steigt, was am AUL-Wert erkennbar ist. Es werden gute bis hervorragende GBP-Werte bei nur wenig beeinträchtigten CRC-Werten erreicht.

Beispiel 6

1 kg Superabsorber-Grundpolymer (HySorb® T 9600 Grundpolymer, nicht oberflä-chennachvernetztes vernetztes Polymer aus Acrylsäure und Natriumacrylat mit Neut-ralisationsgrad 72 mol-%, erhältlich von BASF SE, Ludwigshafen, Deutschland), wurde in einem Mischer vorgelegt. Bei 40 °C und einer Wellendrehzahl von 200 Umdrehungen pro Minute wurde mittels einer Zweistoff-Sprühdüse eine Lösung aus 10 g 1,2-Propandiol, 1 g einer Mischung aus gleichen Gewichtsteilen 2-Hydroxyethyloxazolidinon (HEONON) und 1,3-Propandiol, sowie 26,7 g der Aluminiumsalzlösung Lot # 1099550 aufgesprüht. Anschließend wurde die Wellendrehzahl auf 70 Umdrehungen pro Minute reduziert, die Produkttemperatur binnen 5 bis 10 Minuten auf 190 °C erhöht und anschließend für 80 Minuten gehalten. Während dieser Zeit wurde jeweils nach 20, 30, 40, 50, 60 und 70 Minuten der Mischer kurz angehalten und eine Probe von ca. 10 g Produkt entnommen. Alle Proben sowie die Restmenge des Produkts nach 80 Minuten wurden auf Raumtemperatur abkühlen gelassen. Danach wurde auf Raumtemperatur abkühlen gelassen. Das fertige Produkt wurde durch Siebung auf den Partikelgrößenbereich von 150 $\mu$m bis 710 $\mu$m gewonnen. Die in Tabelle 3 angegebenen Messwerte wurden an den Proben und am so abgesiebten Produkt gemessen.

Beispiel 6a

Beispiel 6 wurde wiederholt, jedoch nur 45 min und ohne Entnahme von Proben auf 190 °C gehalten. Die Messwerte sind in Tabelle 4 angegeben.

Tabelle 2 (in allen Tabellen bedeutet "-": nicht bestimmt)

| Bsp. | 1 | | | 2 | | | 3 | | | 4 | | | 5 | | |
|---|---|---|---|---|---|---|---|---|---|---|---|---|---|---|---|
| Zeit [min] | CRC [g/g] | AUL 0.7psi [g/g] | GBP [Da] | CRC [g/g] | AUL 0.7psi [g/g] | GBP [Da] | CRC [g/g] | AUL 0.7psi [g/g] | GBP [Da] | CRC [g/g] | AUL 0.7psi [g/g] | GBP [Da] | CRC [g/g] | AUL 0.7psi [g/g] | GBP [Da] |
| 20 | 40,9 | 8,4 | - | 33,2 | 22,4 | - | 33,3 | 21,0 | - | 31,2 | 21,9 | - | 31,2 | 21,7 | - |
| 30 | 38,8 | 13,7 | - | 31,5 | 22,1 | - | 30,2 | 21,4 | - | 29,4 | 21,8 | - | 29,8 | 22,0 | - |
| 40 | 35,9 | 19,1 | - | 31,1 | 21,7 | - | 31,0 | 20,9 | - | 28,9 | 21,5 | - | 30,7 | 21,7 | - |
| 50 | 35,1 | 21,7 | - | 29,7 | 21,5 | - | 30,2 | 21 | - | 29,2 | 20,9 | - | 28,7 | 21,1 | - |
| 60 | 36,1 | 22,7 | - | 28,4 | 21,2 | - | 28,8 | 20,6 | - | 26,5 | 20,6 | - | 26,8 | 20,4 | - |
| 70 | 34,9 | 22,9 | - | 26,5 | 20,8 | - | 27,5 | 20,4 | - | 26,8 | 20,3 | - | 28,3 | 20,3 | - |
| 80 | 33,2 | 23,2 | 29,4 | 27,8 | 20,4 | 77,6 | 28,4 | 20,4 | 64,6 | 28,8 | 20,2 | 76,8 | 27,0 | 20,4 | 52,2 |

Tabelle 3

| Bsp. | 6 | | | 7 | | | 8 | | |
|---|---|---|---|---|---|---|---|---|---|
| Zeit [min] | CRC [g/g] | AUL 0.7psi [g/g] | GBP [Da] | CRC [g/g] | AUL 0.7psi [g/g] | GBP [Da] | CRC [g/g] | AUL 0.7psi [g/g] | GBP [Da] |
| 20 | 39,5 | 9,3 | - | 41,1 | 8,4 | - | 41,6 | 7,3 | - |
| 30 | 36,2 | 15,8 | - | 37,1 | 17,5 | - | 39,2 | 10,7 | - |
| 40 | 34,2 | 18,4 | - | 34,6 | 20,5 | - | 37,7 | 15,8 | - |
| 50 | 34,2 | 19,2 | 52,4 | 33,1 | 20,8 | 3,9 | 36,6 | 19,0 | - |
| 60 | 32,6 | 19,2 | - | 35,4 | 20,5 | - | 36,0 | 20,9 | - |
| 70 | 32,2 | 18,9 | - | 31,7 | 20,3 | - | 34,6 | 20,9 | - |
| 80 | 31,0 | 19,4 | 60,3 | 30,9 | 20,0 | 8,0 | 34,0 | 21,0 | 7,3 |

Tabelle 4

| Beispiel | Zeit [min] | CRC [g/g] | AUL 0.7 psi [g/g] | GBP [Da] | anfängliche Farbe | | | Farbe nach 7 Tagen | | | Farbe nach 14 Tagen | | |
|---|---|---|---|---|---|---|---|---|---|---|---|---|---|
| | | | | | L | a | b | L | a | b | L | a | b |
| 6a | 45 | 34,4 | 18,7 | 44,2 | 93,7 | -0,8 | 4,9 | 68,5 | 6,3 | 17,4 | 45 | 11 | 19 |
| 7a | 45 | 35,0 | 20,6 | 3,6 | 90,9 | -0,6 | 4,1 | 59,4 | 8,2 | 20,6 | 31 | 11 | 13 |
| 8 | 80 | 34,0 | 21,0 | 7,3 | 93,1 | -0,6 | 4,4 | 60,7 | 7,9 | 19,9 | 33 | 11 | 14 |

Beispiel 7 (Vergleich)

Beispiel 6 wurde wiederholt, wobei jedoch die 26,7 g Lösung Lot # 1099550 durch 19,5 g Lösung Lot # 1099551 ersetzt wurden. Die erhaltenen Messwerte sind in Tabelle 3 angegeben.

Beispiel 7a (Vergleich)

Beispiel 7 wurde wiederholt, jedoch nur 45 min und ohne Entnahme von Proben auf 190 °C gehalten. Die Messwerte sind in Tabelle 4 angegeben.

Beispiel 8 (Vergleich)

Beispiel 6 wurde wiederholt, wobei jedoch die Lösung Lot # 1099550 durch Lösung Lot # 1099552 ersetzt und die Temperatur nach Aufgabe der Oberflächennachvernetzungslösung auf 180 °C statt 190 °C erhöht wurde.

Die Messwerte zeigen, dass sowohl beim äquivalenten Ersatz von Laktat durch Sulfat (3 Mol Sulfat statt 6 Mol Laktat, d.h. Ersatz unter Berücksichtigung der Wertigkeit) in Beispielen 7 und 7a als auch beim äquimolaren Ersatz (1 Mol Sulfat statt 1 Mol Laktat) in Beispiel 8 sowohl die Permeabilität als auch die Farbstabilität der erfindungsgemäßen Superabsorber nicht erreicht wird. Dies zeigt, dass Löslichkeit von Aluminiumphosphat zwar auch durch Sulfatzusatz erreicht werden kann, die Verwendung dieser Salzkombination in Superabsorbern jedoch Nachteile gegenüber den erfindungsgemäßen Superabsorbern mit sich bringt.

**Patentansprüche**

1. Mit Aluminiumionen komplexierter Superabsorber, wobei die Aluminiumionen in Form einer wässrigen, Aluminiumionen enthaltenden Lösung aufgebracht werden, die sich dadurch auszeichnet, dass sie Aluminiumionen in einem Anteil im Bereich von 0,5 - 15 Gewichts-% (ggf. umgerechnet auf $Al^{3+}$) bezogen auf die Gesamtmasse der Lösung umfasst und weiterhin Anionen der Milchsäure (Laktat-Ionen) und der Phosphorsäure (Phosphat-Ionen) umfasst, wobei der molare Anteil der Laktat-Ionen im Bereich des 0,01 - 2,99-fachen bezogen auf den molaren Anteil an $Al^{3+}$ ist und der molare Anteil der PhosphatIonen im Bereich des 0,01 - 2,99-fachen bezogen auf den molaren Anteil an $Al^{3+}$ ist.

2. Superabsorber nach Anspruch 1, **dadurch gekennzeichnet, dass** die Aluminiumionen in Form einer wässrigen Lösung aufgebracht werden, die weiterhin ein Anion mindestens einer dritten Säure enthält, wobei die dritte Säure bevorzugt ausgewählt ist aus einer Gruppe, die Aminosäuren, Carbonsäuren, Citronensäure, Weinsäure, Äpfelsäure, Oxalsäure, Glykolsäure, Bernsteinsäure, Gluconsäure, Glycin, Essigsäure, Schwefelsäure und/oder Kombinationen umfasst.

3. Superabsorber nach einem der Ansprüche 1 oder 2, **dadurch gekennzeichnet, dass** die Aluminiumionen in Form einer wässrigen Lösung aufgebracht werden, die weiterhin einen Zusatz mindestens eines weiteren Kations enthält, wobei das Kation ausgewählt ist aus einer Gruppe, die Alkaliionen, Erdalkaliionen, Ammonium-Ionen, Kationen eines oder mehrerer Übergangs- oder Seltenerdmetalle oder Kombinationen davon umfasst und besonders bevorzugt ausgewählt ist aus einer Gruppe, die $Na^+$, $K^+$, $Ca^{2+}$, $Mg^{2+}$, $Zr^{2+}$, $NH_4^+$ oder Kombinationen davon umfasst.

4. Superabsorber nach einem der Ansprüche 1 bis 3, **dadurch gekennzeichnet, dass** die Lösung Cluster aufweist, die die theoretische Zusammensetzung $Al^{3+}{}_A(C_3H_5O_3^-)_{x\cdot A}S^M{}_{y\cdot A}(H_2PO_4^-)_{z\cdot A}(OH^-)_{(3A-x\cdot A-M\cdot y\cdot A-z\cdot A)}$ aufweisen, wobei S das Anion einer optional vorhandenen dritten Säure mit der Ladung M ist, x ein Wert im Bereich von 0,01 - 2,99, bevorzugt 0,5 - 2,8, weiter bevorzugt 0,75 - 2,0, meist bevorzugt 1,0 - 1,5 ist, y ein Wert im Bereich von 0 - 2,8, bevorzugt 0 - 2, bevorzugt 0 - 1,25, besonders bevorzugt 0 - 1,0 ist und z ein Wert im Bereich von 0,05 - 2,9, bevorzugt, 0,1 - 2,5, weiter bevorzugt 0,2 - 1,5, besonders bevorzugt 0,3 - 1,25 ist.

5. Superabsorber nach Anspruch 4, **dadurch gekennzeichnet, dass** die Aluminiumionen in Form einer wässrigen Lösung aufgebracht werden, bei der (3A-x·A-M·y·A-z·A) > 0 ist.

6. Superabsorber nach einem der Ansprüche 1 bis 5, **dadurch gekennzeichnet, dass** die Lösung höchstens 5 Gew.-% Sulfationen enthält.

7. Superabsorber nach einem der Ansprüche 1 bis 6, **dadurch gekennzeichnet, dass** er zusätzlich zur Komplexierung mit Aluminiumionen auch mit Nachvernetzern, die kovalente Bindungen mit polaren Gruppen an der Oberfläche der Superabsorberpartikel bilden, an seiner Oberfläche nachvernetzt ist.

8. Superabsorber nach einem der Ansprüche 1 bis 7, **dadurch gekennzeichnet, dass** er mit mindestens 0,008 Gew.-%, und höchstens 0,15 Gew.-%, Aluminium, jeweils berechnet als Metall und bezogen auf die Gesamtmenge des wasserfreien Superabsorbers komplexiert ist.

9. Superabsorber nach Anspruch 4, **dadurch gekennzeichnet, dass** er mit mindestens 0,020 Gew.-%, und höchstens 0,05 Gew.-%, Aluminium, jeweils berechnet als Metall und bezogen auf die Gesamtmenge des wasserfreien Superabsorbers komplexiert ist.

10. Verfahren zur Herstellung eines Superabsorbers durch Polymerisation einer wässrigen Monomerlösung, die

    a) mindestens ein ethylenisch ungesättigtes, säuregruppentragendes Monomer, das wahlweise zumindest teilweise als Salz vorliegt,
    b) mindestens einen Vernetzer,
    c) mindestens einen Initiator,
    d) wahlweise ein oder mehrere mit den unter a) genannten Monomeren copo-lymerisierbare ethylenisch ungesättigte Monomere,
    e) wahlweise ein oder mehrere wasserlösliche Polymere;
    enthält, wobei das Verfahren weiterhin
    Trocknung des erhaltenen Polymerisats,
    wahlweise Mahlung des getrockneten Polymerisats und Siebung des gemahlenen Polymerisats,
    wahlweise Oberflächennachvernetzung des getrockneten und gegebenenfalls gemahlenen und gesiebten Polymerisats, und
    Zugabe einer wässrigen, Aluminiumionen enthaltenden Lösung, die sich dadurch auszeichnet, dass sie Aluminiumionen in einem Anteil im Bereich von 0,5 - 15 Gewichts-% (ggf. umgerechnet auf $Al^{3+}$) bezogen auf die Gesamtmasse der Lösung umfasst und weiterhin Anionen der Milchsäure (Laktat-Ionen) und der Phosphorsäure (Phosphat-Ionen) umfasst, wobei der molare Anteil der Laktat-Ionen im Bereich des 0,01 - 2,99-fachen bezogen auf den molaren Anteil an $Al^{3+}$ ist und der molare Anteil der Phosphat-Ionen im Bereich des 0,01 - 2,99-fachen bezogen auf den molaren Anteil an $Al^{3+}$ ist, umfasst.

**11.** Verfahren nach Anspruch 11, **dadurch gekennzeichnet, dass** die Aluminiumionen enthaltende Lösung höchstens 5 Gew.-% Sulfationen enthält.

**12.** Artikel zur Absorption von Flüssigkeiten, enthaltend einen in den Ansprüchen 1 bis 5 definierten Superabsorber.

**13.** Verfahren zur Herstellung von Artikeln zur Absorption von Flüssigkeit, **dadurch gekennzeichnet, dass** man bei der Herstellung der Artikel ihnen einen in den Ansprüchen 1 bis 5 definierten Superabsorber zusetzt.

**Claims**

**1.** A superabsorbent complexed with aluminium ions, where the aluminium ions are applied in the form of an aqueous solution comprising aluminium ions, which has the feature that it comprises aluminium ions in a proportion within the range of 0.5%-15% by weight (optionally converted to $Al^{3+}$), based on the total mass of the solution, and further comprises anions of lactic acid (lactate ions) and phosphoric acid (phosphate ions), where the molar proportion of the lactate ions is within the range of 0.01-2.99 times the molar amount of $Al^{3+}$ and the molar proportion of the phosphate ions is within the range of 0.01-2.99 times the molar amount of $Al^{3+}$.

**2.** The superabsorbent according to claim 1, wherein the aluminium ions are applied in the form of an aqueous solution further comprising an anion of at least one third acid, where the third acid is preferably selected from a group comprising amino acids, carboxylic acids, citric acid, tartaric acid, malic acid, oxalic acid, glycolic acid, succinic acid, gluconic acid, glycine, acetic acid, sulfuric acid and/or combinations.

**3.** The superabsorbent according to either of claims 1 and 2, wherein the aluminium ions are applied in the form of an aqueous solution further comprising an addition of at least one further cation, where the cation is selected from a group comprising alkali metal ions, alkaline earth metal ions, ammonium ions, cations of one or more transition metals or rare earth metals or combinations thereof and is more preferably selected from a group comprising $Na^+$, $K^+$, $Ca^{2+}$, $Mg^{2+}$, $Zr^{2+}$, $NH_4^+$ or combinations thereof.

**4.** The superabsorbent according to any of claims 1 to 3, wherein the solution includes clusters having the theoretical composition $Al^{3+}{}_A (C_3H_5O_3^-)_{x \cdot A} S^{M-}{}_{y \cdot A} (H_2PO_4^-)_{z \cdot A} (OH^-)_{(3A-x \cdot A-M \cdot y \cdot A-z \cdot A)}$ where S is the anion of an optionally present third acid having charge M, x is a value within the range of 0.01-2.99, preferably 0.5-2.8, further preferably 0.75-2.0, most preferably 1.0-1.5, y is a value within the range of 0-2.8, preferably 0-2, preferably 0-1.25, more preferably 0-1.0, and z is a value within the range of 0.05-2.9, preferably 0.1-2.5, further preferably 0.2-1.5, more preferably 0.3-1.25.

**5.** The superabsorbent according to claim 4, wherein the aluminium ions are applied in the form of an aqueous solution in which $(3A-x \cdot A-M \cdot y \cdot A-z \cdot A) > 0$.

**6.** The superabsorbent according to any of claims 1 to 5, wherein the solution comprises not more than 5% by weight of sulfate ions.

**7.** The superabsorbent according to any of claims 1 to 6, which, as well as having been complexed with aluminium ions, has also been surface postcrosslinked with postcrosslinkers that form covalent bonds with polar groups at the surface of the superabsorbent particles.

**8.** The superabsorbent according to any of claims 1 to 7, which has been complexed with at least 0.008% by weight and at most 0.15% by weight of aluminium, calculated in each case as the metal and based on the total amount of the anhydrous superabsorbent.

**9.** The superabsorbent according to claim 4, which has been complexed with at least 0.020% by weight and at most 0.05% by weight of aluminium, calculated in each case as the metal and based on the total amount of the anhydrous superabsorbent.

10. A process for producing a superabsorbent by polymerizing an aqueous monomer solution comprising

a) at least one ethylenically unsaturated monomer which bears acid groups and is optionally at least partly in salt form,
b) at least one crosslinker,
c) at least one initiator,
d) optionally one or more ethylenically unsaturated monomers copolymerizable with the monomers mentioned under a),
e) optionally one or more water-soluble polymers; the process further comprising
drying of the resulting polymer,
optionally grinding of the dried polymer and sieving of the ground polymer,
optionally surface postcrosslinking of the dried and optionally ground and sieved polymer, and
adding an aqueous solution comprising aluminium ions, which has the feature that it comprises aluminium ions in a proportion within the range of 0.5%-15% by weight (optionally converted to $Al^{3+}$), based on the total mass of the solution, and further comprises anions of lactic acid (lactate ions) and phosphoric acid (phosphate ions), where the molar proportion of the lactate ions is within the range of 0.01-2.99 times the molar amount of $Al^{3+}$ and the molar proportion of the phosphate ions is within the range of 0.01-2.99 times the molar amount of $Al^{3+}$.

11. The process according to claim 11, wherein the solution comprising aluminium ions comprises not more than 5% by weight of sulfate ions.

12. An article for absorption of fluids, comprising a superabsorbent defined in claims 1 to 5.

13. A process for producing articles for absorption of fluid, wherein the production of the articles involves addition of a superabsorbent defined in claims 1 to 5.


**Revendications**

1. Superabsorbant complexé avec des ions aluminium, dans lequel les ions aluminium sont appliqués sous la forme d'une solution aqueuse contenant des ions aluminium, qui est **caractérisée en ce qu'**elle contient des ions aluminium en une proportion dans la plage de 0,5 à 15 % en poids (converti le cas échéant en $Al^{3+}$) par rapport à la masse totale de la solution et comprend en outre des anions de l'acide lactique (ions lactate) et de l'acide phosphorique (ions phosphate), la proportion molaire des ions lactate étant dans la plage de 0,01 à 2,99 fois par rapport à la proportion molaire d'$Al^{3+}$ et la proportion molaire des ions phosphate étant dans la plage de 0,01 à 2,99 fois par rapport à la proportion molaire d'$Al^{3+}$.

2. Superabsorbant selon la revendication 1, **caractérisé en ce que** les ions aluminium sont appliqués sous la forme d'une solution aqueuse qui contient en outre un anion d'au moins un troisième acide, le troisième acide étant préférablement choisi dans un groupe comprenant des acides aminés, des acides carboxyliques, l'acide citrique, l'acide tartrique, l'acide malique, l'acide oxalique, l'acide glycolique, l'acide succinique, l'acide gluconique, la glycine, l'acide acétique, l'acide sulfurique et/ou des combinaisons correspondantes.

3. Superabsorbant selon l'une quelconque des revendications 1 et 2, **caractérisé en ce que** les ions aluminium sont appliqués sous la forme d'une solution aqueuse qui contient en outre un ajout d'au moins un autre cation, ledit cation étant choisi dans un groupe qui comprend des ions alcalins, des ions alcalino-terreux, des ions ammonium, des cations d'un ou plusieurs métaux de transition ou de terres rares ou des combinaisons correspondantes, et particulièrement préférablement étant choisi dans un groupe qui comprend $Na^+$, $K^+$, $Ca^{2+}$, $Mg^{2+}$, $Zr^{2+}$, $NH_4^+$ ou des combinaisons correspondantes.

4. Superabsorbant selon l'une quelconque des revendications 1 à 3, **caractérisé en ce que** la solution présente des clusters ayant la composition théorique $Al^{3+}_A(C_3H_5O_3^-)_{x \cdot A}S^{M-}_{y \cdot A}(H_2PO_4^-)_{z \cdot A}(OH^-)_{(3A-x \cdot M \cdot y \cdot A-z \cdot A)}$, où S est l'anion d'un troisième acide éventuellement présent doté de la charge M, x est une valeur dans la plage de 0,01 à 2,99, préférablement de 0,5 à 2,8, plus préférablement de 0,75 à 2,0, le plus préférablement de 1,0 à 1,5, y est une valeur dans la plage de 0 à 2,8, préférablement de 0 à 2, préférablement de 0 à 1,25, particulièrement préférablement de 0 à 1,0 et z est une valeur dans la plage de 0,05 à 2,9, préférablement de 0,1 à 2,5, plus préférablement de 0,2 à 1,5, particulièrement préférablement de 0,3 à 1,25.

**5.** Superabsorbant selon la revendication 4, **caractérisé en ce que** les ions aluminium sont appliqués sous la forme d'une solution aqueuse dans laquelle **(3A-X·A-M·y·A-Z·A) > 0**.

**6.** Superabsorbant selon l'une quelconque des revendications 1 à 5, **caractérisé en ce que** la solution contient au plus 5 % en poids d'ions sulfate.

**7.** Superabsorbant selon l'une quelconque des revendications 1 à 6, **caractérisé en ce que**, en plus d'être complexé par des ions aluminium, il est également post-réticulé au niveau de sa surface par des agents de post-réticulation qui forment des liaisons covalentes avec des groupes polaires à la surface des particules de superabsorbant.

**8.** Superabsorbant selon l'une quelconque des revendications 1 à 7, **caractérisé en ce qu'**il est complexé avec au moins 0,008 % en poids, et au plus 0,15 % en poids, d'aluminium, à chaque fois calculé en tant que métal et par rapport à la quantité totale du superabsorbant anhydre.

**9.** Superabsorbant selon la revendication 4, **caractérisé en ce qu'**il est complexé avec au moins 0,020 % en poids, et au plus 0,05 % en poids, d'aluminium, à chaque fois calculé en tant que métal et par rapport à la quantité totale du superabsorbant anhydre.

**10.** Procédé de préparation d'un superabsorbant par polymérisation d'une solution aqueuse de monomères qui contient

a) au moins un monomère éthyléniquement insaturé portant des groupes de type acide, qui est éventuellement présent au moins partiellement en tant que sel,
b) au moins un agent de réticulation,
c) au moins un initiateur,
d) éventuellement un ou plusieurs monomères éthyléniquement insaturés copolymérisables avec les monomères mentionnés sous a),
e) éventuellement un ou plusieurs polymères solubles dans l'eau ;
le procédé comprenant en outre
le séchage du polymérisat obtenu,
éventuellement le broyage du polymérisat séché et le tamisage du polymérisat broyé,
éventuellement la post-réticulation de surface du polymérisat séché et éventuellement broyé et tamisé, et l'ajout d'une solution aqueuse contenant des ions aluminium, qui est **caractérisée en ce qu'**elle contient des ions aluminium en une proportion dans une plage comprise entre 0,5 et 15 % en poids (converti le cas échéant en $Al^{3+}$) par rapport à la masse totale de la solution et comprend en outre des anions de l'acide lactique (ions lactate) et de l'acide phosphorique (ions phosphate), la proportion molaire des ions lactate étant dans la plage de 0,01 à 2,99 fois par rapport à la proportion molaire d'$Al^{3+}$ et la proportion molaire des ions phosphate étant dans la plage de 0,01 à 2,99 fois par rapport à la proportion molaire d'$Al^{3+}$.

**11.** Procédé selon la revendication 11, **caractérisé en ce que** la solution contenant des ions aluminium contient au plus 5 % en poids d'ions sulfate.

**12.** Article pour l'absorption de liquides, contenant l'un des superabsorbants définis dans les revendications 1 à 5.

**13.** Procédé de préparation d'articles pour l'absorption de liquide, **caractérisé en ce qu'**on ajoute aux articles l'un des superabsorbants définis dans les revendications 1 à 5 lors de la préparation des articles.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9848857 A1
- WO 0174913 A1
- US 6620889 B1
- WO 2006111402 A2
- WO 2005108472 A1
- WO 2004113452 A1
- WO 2013156281 A1
- WO 2010108875 A1
- WO 2012045705 A1
- WO 2013156330 A1
- WO 2009080611 A2
- WO 0055245 A1
- WO 2006058682 A1
- WO 2009060062 A1
- WO 2010012762 A2
- EP 1199315 A2
- WO 9918067 A1
- WO 2004084962 A1
- JP 5086251 A
- EP 781804 A2
- EP 668080 A2
- US 20050085604 A1
- US 20050272600 A1
- EP 2112172 A1
- US 20090275470 A1
- WO 2006109882 A1
- WO 2013144026 A1
- EP 2163302 A1
- WO 2002055469 A1
- WO 2003078378 A1
- WO 2004035514 A1
- EP 530438 A1
- EP 547847 A1
- EP 559476 A1
- EP 632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 200232962 A2
- WO 200138402 A1
- EP 955086 A2
- DE 3825366 A1
- US 6241928 B
- EP 445619 A2
- DE 19846413 A1
- EP 457660 A1
- EP 348180 A1
- EP 816383 A1
- WO 9640427 A1
- US 4020256 A
- US 20020193546 A1
- DE 3519013 A1
- DE 102005044035 A1
- WO 2007093531 A1
- WO 2008086976 A1
- WO 2009027356 A1
- WO 0294328 A2
- WO 0294329 A1
- EP 0534228 A1
- EP 1191051 A2
- US 20030181115 A
- US 20040019342 A
- EP 0377199 A2
- EP 0445641 A1
- US 5026806 A
- EP 0655465 A1
- EP 0377191 A2
- EP 0640330 A1
- US 20050256757 A1

**In der Beschreibung aufgeführte Nicht-Patentliteratur**

- Modern Superabsorbent Polymer Technology. J. Wiley & Sons, 1997
- **ZERSTÄUBEN VON FLÜSSIGKEITEN.** Reihe Kontakt & Studium. Expert-Verlag, vol. 660
- **THOMAS RICHTER.** Zerstäubungstechnik. Springer-Verlag, 2004
- **GÜNTER WOZNIAK.** *VDI-Reihe,* 2002
- CIELAB-Verfahren. Hunterlab, 1996, vol. 8, 1-4